(19) Europäisches Patentamt
European Patent Office
Office européen des brevets

(11) EP 0 693 496 A1

(12) DEMANDE DE BREVET EUROPEEN

(43) Date de publication: 24.01.1996 Bulletin 1996/04

(51) Int Cl.⁶: C07F 9/6561, A61K 31/675, C07F 9/553, C07F 9/6539, C07F 9/40

(21) Numéro de dépôt: 95401699.4

(22) Date de dépôt: 18.07.1995

(84) Etats contractants désignés: AT BE CH DE DK ES FR GB GR IE IT LI LU NL PT SE

(30) Priorité: 19.07.1994 FR 9408912

(71) Demandeur: ROUSSEL UCLAF
F-93230 Romainville (FR)

(72) Inventeurs:
- Aszodi, Jozsef
F-77340 Pontault Combault (FR)
- Fauveau, Patrick
F-93190 Livry Gargan (FR)

(74) Mandataire: Vieillefosse, Jean-Claude et al
F-93235 Romainville Cédex (FR)

# (54) Nouvelles céphalosporines comportant en position 7 un radical benzyloxyimino substitué, leur procédé de préparation, leur application comme médicaments

(57) L'invention a pour objet les produits de formule générale (I) :

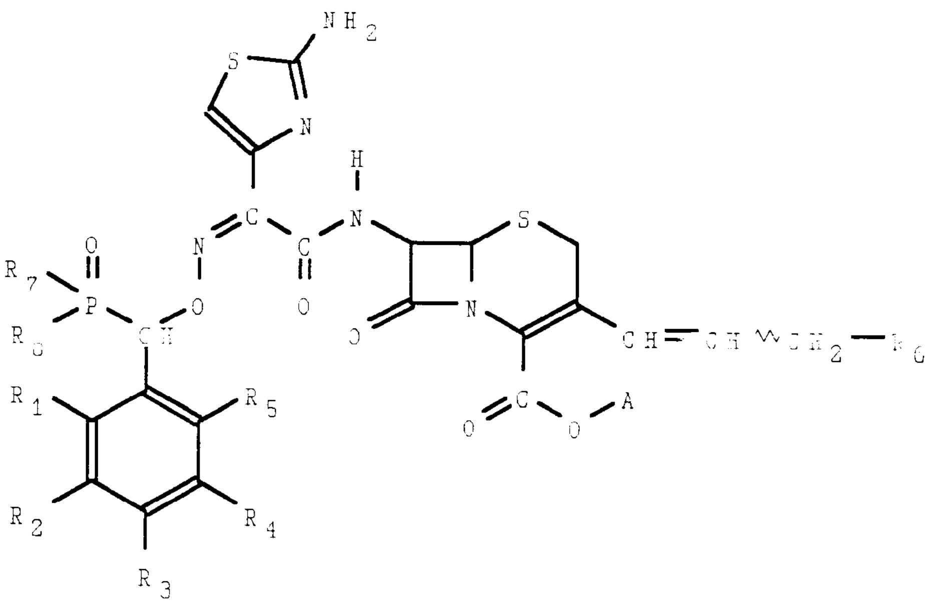

(I)

isomère syn, dans laquelle :

$R_1$, $R_2$, $R_3$ et $R_5$ représentent un atome d'hydrogène ou d'halogène ou un radical hydroxy, alkyl, alkyloxy, mercapto, alkylthio, nitro, cyano, amino, alkylamino, dialkylamino, carbamoyle, (alkylamino) carbonyle, (dialkylamino) carbonyle, carboxy, alcoxycarbonyle, acyloxy, amino ou alkylamino-sulfonyle,

$R_4$ représente un radical hydroxy ou acyloxy,

$R_7$ représente un radical alkyle, hydroxy, alkyloxy ou phényl,

$R_8$ représente un radical hydroxy ou alkyloxy

ou $R_7$ et $R_8$ représentent ensemble un radical alkylène dioxy, A représente un atome d'hydrogène, un équivalent de métal alcalin, alcalino-terreux, de magnésium, d'ammonium ou d'une base organique aminée, ou bien $CO_2A$ représente $CO_2^{\ominus}$, et $-CH_2R_6$ peut être en position E Ou Z.

Ces produits possèdent d'intéressantes propriétés pharmacologiques qui justifient leur emploi comme médicaments.

EP 0 693 496 A1

## Description

La présente invention concerne de nouvelles céphalosporines comportant sur la chaîne latérale en position 7, un radical benzyloxyimino substitué, leur procédé de préparation, leur application comme médicaments, les compositions les renfermant et les nouveaux intermédiaires obtenus.

L'invention a pour objet les produits de formule générale (I) :

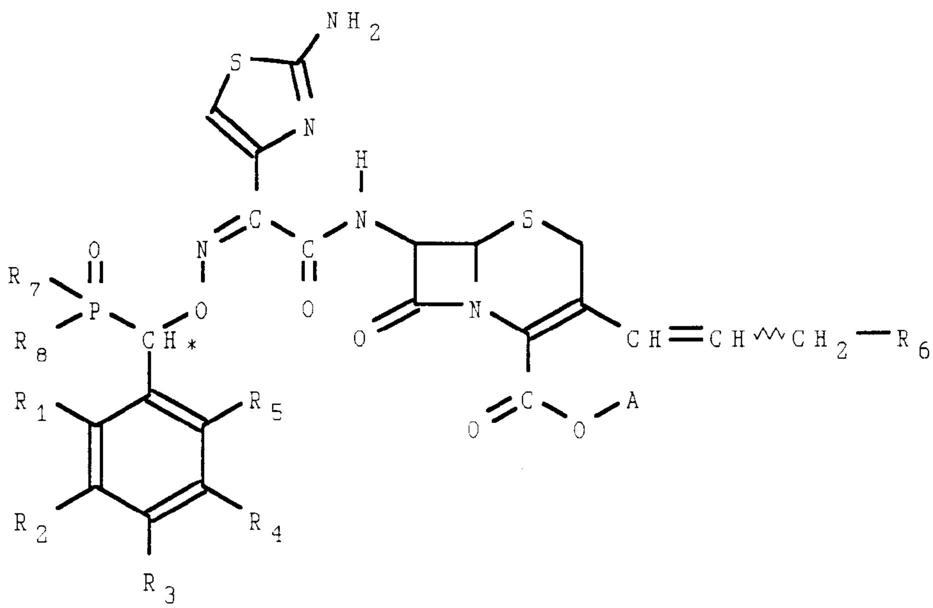

(I)

isomère syn, sous forme (*) (R) ou (S) ou d'un mélange (R,S), sous forme de sels internes ou de sels avec les acides minéraux ou organiques ou avec les bases, formule dans laquelle :

$R_1$, $R_2$, $R_3$ et $R_5$ identiques ou différents représentent un atome d'hydrogène, un atome d'halogène ou un des radicaux choisis parmi le radical hydroxy, alkyle renfermant de 1 à 4 atomes de carbone, éventuellement substitué par un ou plusieurs atomes d'halogène, alkyloxy renfermant de 1 à 4 atomes de carbone, mercapto, alkylthio renfermant de 1 à 4 atomes de carbone, nitro, cyano, amino, alkylamino renfermant de 1 à 4 atomes de carbone, dialkylamino renfermant de 2 à 8 atomes de carbone, carbamoyle, (alkylamino) carbonyle renfermant de 2 à 5 atomes de carbone, (dialkylamino) carbonyle renfermant de 3 à 9 atomes de carbone, carboxy, alcoxycarbonyle renfermant de 2 à 5 atomes de carbone, acyloxy renfermant de 1 à 8 atomes de carbone,

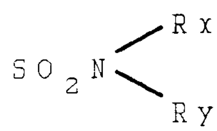

dans lequel Rx et Ry identiques ou différents représentent un atome d'hydrogène ou un radical alkyle renfermant de 1 à 4 atomes de carbone,

$R_4$ représente un radical hydroxy ou un radical acyloxy renfermant de 1 à 8 atomes de carbone,

$R_7$ représente un radical alkyle renfermant de 1 à 4 atomes de carbone, un radical hydroxy, un radical alkyloxy renfermant de 1 à 4 atomes de carbone ou un radical phényl,

$R_8$ représente un radical hydroxy ou un radical alkyloxy renfermant de 1 à 4 atomes de carbone,

ou $R_7$ et $R_8$ représentent ensemble un radical alkylène dioxy renfermant de 2 à 8 atomes de carbone,

A représente un atome d'hydrogène, un équivalent de métal alcalin, alcalino-terreux, de magnésium, d'ammonium ou d'une base organique aminée ou A représente le reste d'un groupement ester facilement clivable, ou bien $CO_2A$ représente $CO_2^{\ominus}$, le trait ondulé signifie aue le groupement $CH_2R_6$ peut se trouver dans la position E ou Z et $R_6$ représente sous forme d'ammonium quaternaire, un des radicaux suivants :

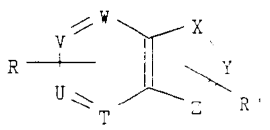 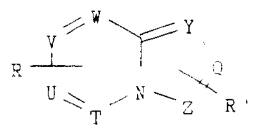 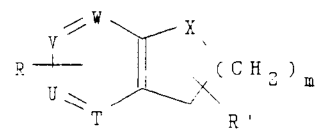 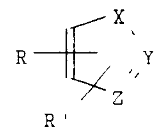

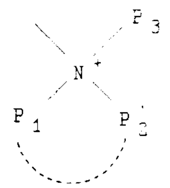 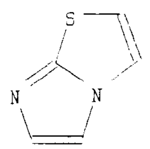 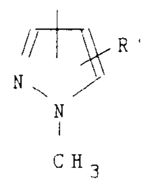 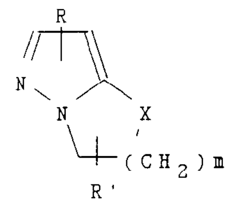

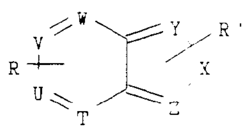 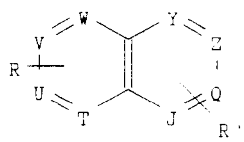 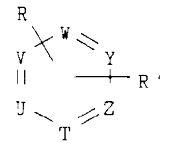 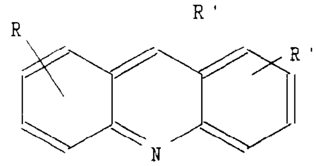

dans lesquels X représente $CH_2$, NH, O ou S ;

Q, J, Y, T, U, V, W et Z identiques ou différents représentent indépendamment les uns des autres CH ou N, étant entendu que chacun de ces radicaux cycliques contient de 1 à 5 hétéroatomes, qu'au moins un de ces hétéroatomes est l'atome d'azote et que ces radicaux cycliques peuvent être substitués par un ou plusieurs radicaux R ou R' ;

R et R', identiques ou différents, représentent un atome d'hydrogène, un radical alkyle renfermant de 1 à 4 atomes de carbone, un radical alkyloxy renfermant de 1 à 4 atomes de carbone, un atome d'halogène, un radical cyano, un radical $CO_2$-$Q_1$, CO-$NQ_1(Q_2)$, $NQ_1(Q_2)$, $SO_2$-$NQ_1(Q_2)$, CS-$NH_2$, NH-CO-$Q_1$, CH=N-OH, CH=N-O-$Q_1$, $CH_2$-CN, $CH_2$-S-$Q_1$, $SQ_1$, dans lesquels $Q_1$ et $Q_2$, identiques ou différents, représentent un atome d'hydrogène ou un radical alkyle renfermant de 1 à 4 atomes de carbone, $P_1$, $P_2$ et $P_3$, identiques ou différents, représentent un radical alkyle renfermant de 1 à 4 atomes de carbone, éventuellement substitué par un des substituants indiqués ci-dessus pour R et R', ou encore, pour l'un d'entre eux, par un radical hydroxy, le trait pointillé indique que $P_1$ et $P_2$ peuvent éventuellement former avec l'atome d'azote auquel ils sont liés, un hétérocycle à 5 ou 6 chaînons et m = 1, 2 ou 3.

Par radical alkyle renfermant de 1 à 4 atomes de carbone, on entend les radicaux, méthyle, éthyle, propyle, isopropyle, butyle, isobutyle, sec-butyle ou tert-butyle.

Par radical alkyloxy renfermant de 1 à 4 atomes de carbone, on entend les radicaux, méthoxy, éthoxy, propoxy, isopropoxy, butoxy, isobutoxy, sec-butoxy ou tert-butoxy.

Par radical alkylthio renfermant de 1 à 4 atomes de carbone, on entend les radicaux, méthylthio, éthylthio, propylthio, isopropylthio, butylthio, isobutylthio, sec-butylthio ou tert-butylthio.

Par radical alkylamino renfermant de 1 à 4 atomes de carbone, on entend les radicaux, méthylamino, éthylamino, propylamino, isopropylamino, butylamino, isobutylamino, sec-butylamino ou tert-butylamino.

Par radical dialkylamino renfermant de 2 à 8 atomes de carbone, on entend notamment les radicaux, diméthylamino, diéthylamino, dipropylamino, diisopropylamino, dibutylamino, diisobutylamino, éthyl méthylamino, propyl méthylamino, butyl méthylamino, propyl éthylamino.

Par radical (alkylamino) carbonyle renfermant de 2 à 5 atomes de carbone, on entend notamment les radicaux, (méthylamino) carbonyle, (éthylamino) carbonyle, (propylamino) carbonyle, (isopropylamino) carbonyle, (butylamino) carbonyle.

Par radical alkoxycarbonyle renfermant de 2 à 5 atomes de carbone, on entend notamment les radicaux, méthoxycarbonyle, éthoxycarbonyle.

Par radical (dialkylamino) carbonyle renfermant de 3 à 9 atomes de carbone, on entend notamment les radicaux, (diméthylamino) carbonyle, (diéthylamino) carbonyle, (dipropylamino) carbonyle.

Par radical acyloxy renfermant de 1 à 8 atomes de carbone, on entend notamment les radicaux acétoxy, propionyloxy ou benzoyloxy.

Par radical alkylènedioxy renfermant de 2 à 8 atomes de carbone, on entend un radical de type linéaire ou de type ramifié, notamment un radical éthylènedioxy, triméthylènedioxy, 1,2-diméthyléthylènedioxy ou encore triméthylènedioxy substitué par un ou deux radicaux méthyle en particulier géminés.

Par atome d'halogène, on entend atome de fluor, chlore, brome ou iode.

Lorsque $P_1$ et $P_2$ forment un hétérocycle avec l'atome d'azote auquel ils sont liés, il peut s'agir d'une pyrrolidine, d'une morpholine ou d'une pipéridine.

Lorsque $R_4$ représente un radical acyloxy, il s'agit notamment des radicaux acétoxy, propionyloxy ou benzoyloxy.

Parmi les valeurs de A on peut citer un équivalent de sodium, de potassium, de lithium, de calcium, de magnésium

ou d'ammonium. On peut citer, un équivalent d'une base organique, par exemple la méthylamine, la propylamine, la triméthylamine, la diéthylamine, la triéthylamine, la N,N-diméthyléthanolamine, le tris[(hydroxyméthyl) amino] méthane, l'éthanolamine, la pyridine, la picoline, la dicyclohexylamine, la morpholine, la benzylamine, la procaïne, la lysine, l'arginine, l'histidine, la N-méthylglucamine.

On peut citer entre autres restes de groupements ester facilement clivables que peut représenter A, les groupements méthoxyméthyle, éthoxyméthyle, isopropyloxyméthyle, alphaméthoxy éthyle, alpha-éthoxy éthyle, méthylthiométhyle, éthylthiométhyle, isopropylthiométhyle, pivaloyloxyméthyle, acétoxyméthyle, propionyloxyméthyle, butyryloxyméthyle, isobutyryloxyméthyle, valéryloxyméthyle, isovaléryloxyméthyle, tert-butylcarbonyloxyméthyle, hexadécanoyloxyméthyle, propionyloxyéthyle, isovaléryloxyéthyle, 1-acétyloxyéthyle, 1-propionyloxyéthyle, 1-butyryloxyéthyle, 1-tert-butylcarbonyloxyéthyle, 1-acétyloxypropyle, 1-hexadécanoyloxyéthyle, 1-propionyloxypropyle, 1-méthoxycarbonyloxyéthyle, méthoxycarbonyloxyméthyle, 1-acétyloxybutyle, 1-acétyloxyhexyle, 1-acétyloxyheptyle, phtalidyle, 5,6-diméthoxyphtalidyle, tert-butylcarbonylméthyle, allyle, 2-chloroallyle, méthoxycarbonylméthyle, benzyle ou tert-butyle.

On peut encore citer entre autres restes de groupements esters que peut représenter A, les groupements méthoxyéthoxyméthyle, diméthylaminoéthyle, cyanométhyle, tert-butoxycarbonylméthyle, 2,2-éthylènedioxyéthyle, cyanoéthyle, 2,2-diméthoxyéthyle ; 2-chloroéthoxyméthyle, 2-hydroxyéthoxyéthyle, 2,3-époxypropyle, 3-diméthylamino, 2-hydroxypropyle, 2-hydroxyéthyle, 2-méthylaminoéthoxyméthyle, 2-aminoéthoxyméthyle, 3-méthoxy 2,4-thiadiazol-5-yle, 2-tétrahydropyrannyle, 1-méthoxy 1-méthyl éthyle, 2-hydroxy 1-méthyl éthyle, isopropyle ; carbamoylméthyle, chlorométhyle, 2-chloroéthyle, acétylméthyle, 2-méthylthioéthyle ou thiocyanatométhyle.

On peut encore citer entre autres restes de groupements esters que peut représenter A, les groupements 2-chloro 1-acétyloxyéthyle, 2-bromo 1-acétyloxyéthyle, 2-fluoro 1-acétyloxyéthyle, 2-méthoxy 1-acétyloxyéthyle, 2-méthyl 1-acétyloxypropyle, 1-méthyl 1-acétyloxyéthyle, 1-méthoxyacétyloxyéthyle, 1-acétylcarbonyloxyéthyle, 1-hydroxyacétyloxyéthyle, 1-formylcarbonyloxyéthyle, 1-(2-thiényl) carbonyloxyéthyle, 1-(2-furyl) carbonyloxyéthyle, 1-(5-nitro 2-furyl) carbonyloxyéthyle, 1-(2-pyrrolyl) carbonyloxyéthyle, 1-(propionyloxycarbonyloxy) éthyle, 1-(propyloxycarbonyloxy) éthyle, 1-(isopropyloxycarbonyloxy) éthyle, 1-(méthoxyéthoxycarbonyloxy) éthyle, 1-(allyloxycarbonyloxy) éthyle, isopropyloxycarbonyl méthyle, 1-[(2,3-époxy propyl) oxycarbonyloxy] éthyle, 1-[(2-furyl) méthyloxycarbonyloxy] éthyle, 1-(2-fluoro éthyl) oxycarbonyloxyéthyle, 1-(méthoxycarbonyloxy) propyle, 1-(méthoxycarbonyloxy) 1-méthyl éthyle, (méthoxycarbonyloxy) chlorométhyle, 1-(méthoxycarbonyloxy) 2-chloroéthyle, 1-(méthoxycarbonyloxy) 2-méthoxy éthyle, 1-(méthoxycarbonyloxy) allyle, cu un reste :

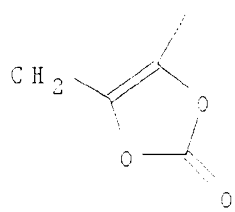

Les produits de formule (I) peuvent également se présenter sous forme de sel interne pur, sous forme salifiée ou sous forme associée aux acides de la solution, ou encore sous forme de sels de bases au niveau des autres radicaux carboxy ou au niveau du ou des radicaux hydroxy portés par l'atome de phosphore.

Parmi les acides avec lesquels on peut salifier les produits de formule (I), on peut citer entre autres, les acides acétique, trifluoroacétique, maléïque, tartrique, méthanesulfonique, benzènesulfonique, para-toluènesulfonique, phosphorique, sulfurique, chlorhydrique, bromhydrique, iodhydrique.

Parmi les bases avec lesquelles on peut salifier les produits de formule (I), on peut citer, entre autres, les bases correspondant aux sels mentionnés plus haut.

Dans un mode préféré de l'invention $CO_2A$ représente $CO_2^{\ominus}$.

L'expression "sous forme d'ammonium quaternaire" indique que le radical $R_6$ est lié par le ou l'un des atomes d'azote qu'il comporte.

L'invention a particulièrement pour objet les produits de formule générale (I) telle que définie ci-dessus dans laquelle $R_6$ représente un des radicaux suivants :

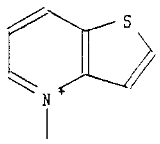 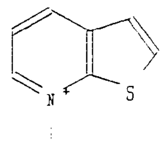 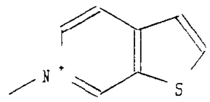 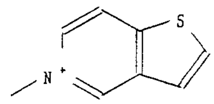

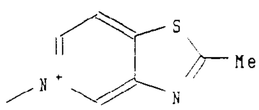 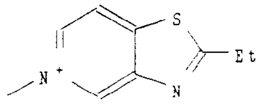 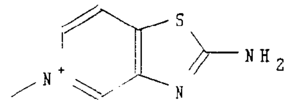

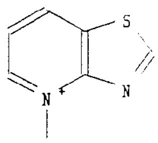 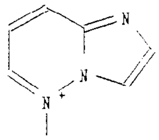 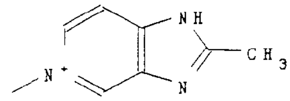

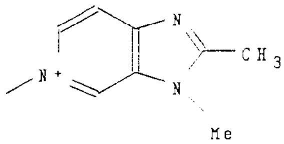 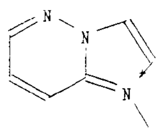 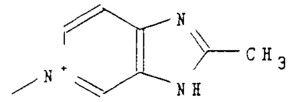

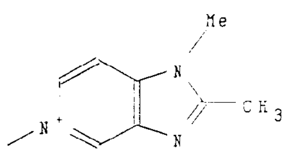 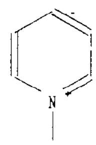 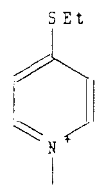 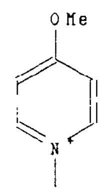

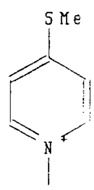 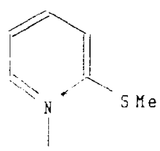 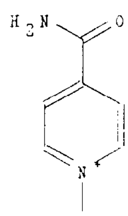 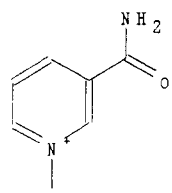

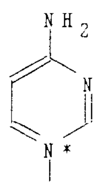 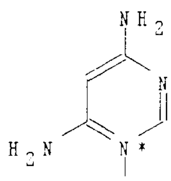 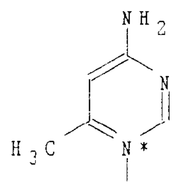 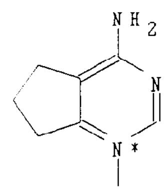

ou encore l'un des radicaux suivants :

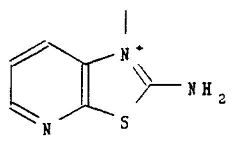 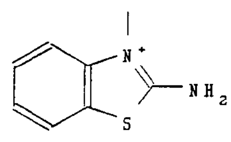 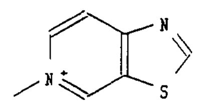

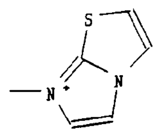 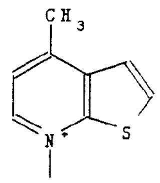 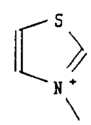 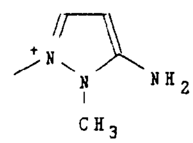

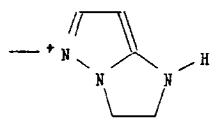 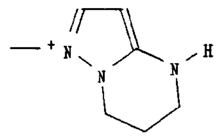

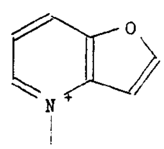 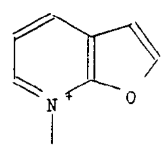 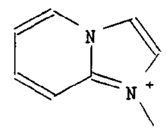 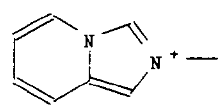

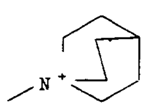 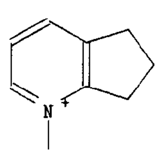 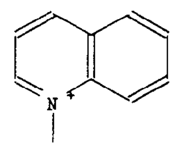

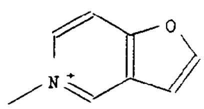 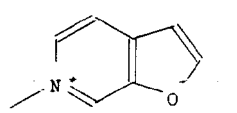

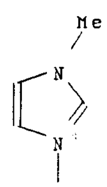 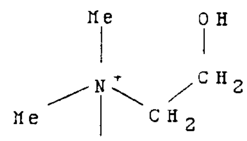 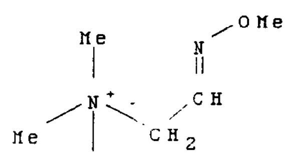

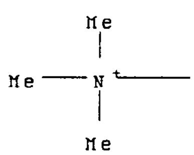 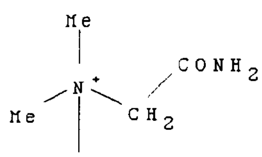 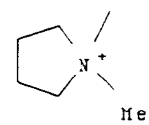

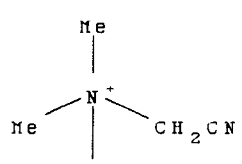 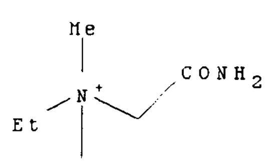 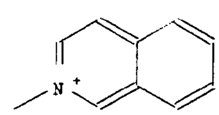

L'invention a plus particulièrement pour objet les produits de formule générale (I) telle que définie ci-dessus dans laquelle $R_6$ représente le radical quinoléinium, isoquinoléinium, 4-(méthylthio) pyridinium, thièno[2,3-b]pyridinium, 1-méthyl pyrrolidinium, N-méthyl N-éthyl N-(2-amino 2-oxoéthyl) aminium, imidazo(1,2-a)pyridinium ou le radical 6,7-dihydro-5H-pyrindinium, ceux dans laquelle $R_3$ et $R_4$ représentent chacun un radical hydroxy et ceux dans laquelle $R_2$ et

$R_5$ représentent chacun un atome de chlore ou de fluor, ceux dans laquelle $R_1$ et $R_2$ représentent chacun un atome de fluor et ceux dans laquelle $R_2$ représente un radical méthoxy et l'un de $R_1$ ou $R_5$ représente un atome de chlore.

L'invention a tout particulièrement pour objet les produits dont les noms suivent :

- le sel interne de (6R-(3-(E) 6alpha, 7béta-(Z))) 1-(3-(7-(((2-amino 4-thiazolyl) ((éthoxyméthylphosphinyl (3,4-dihydroxyphényl) méthoxy) imino) acétyl) amino) 2-carboxy 8-oxo 5-thia 1-azabicyclo[4,2,0)oct-2-èn-3-yl) 2-propényl) quinoléinium,
- le sel interne de (6R-(3-(E) 6alpha, 7béta-(Z))) 7-(3-(7-(((2-amino 4-thiazolyl) ((éthoxyméthylphosphinyl (3,4-dihydroxyphényl) méthoxy) imino) acétyl) amino) 2-carboxy 8-oxo 5-thia 1-azabicyclo[4,2,0]oct-2-èn-3-yl) 2-propényl) thiéno(2,3-b)pyridinium,
- le sel interne de (6R-(3-(E) 6alpha, 7béta-(Z))) 7-(3-(7-(((2-amino 4-thiazolyl) ((hydroxyméthylphosphinyl (3,4-dihydroxyphényl) méthoxy) imino) acétyl) amino) 2-carboxy 8-oxo 5-thia 1-azabicyclo[4,2,0]oct-2-èn-3-yl) 2-propényl) thiéno(2,3-b)pyridinium,
- le sel interne de (6R-(3-(E) 6alpha, 7béta-(Z))) 7-(3-(7-(((2-amino 4-thiazolyl) (((2,5-dichloro 3,4-dihydroxyphényl) (diéthoxyphosphinyl) méthoxy) imino) acétyl) amino) 2-carboxy 8-oxo 5-thia 1-azabicyclo[4,2,0]oct-2-èn-3-yl) 2-propényl) thiéno(2,3-b)pyridinium,
- le sel interne de (6R-(3-(E) 6alpha, 7béta-(Z))) 7-(3-(7-(((2-amino 4-thiazolyl) (((2,5-dichloro 3,4-dihydroxyphényl) phosphonométhoxy) imino) acétyl) amino) 2-carboxy 8-oxo 5-thia 1-azabicyclo[4,2,0]oct-2-èn-3-yl) 2-propényl) thiéno(2,3-b)pyridinium,
- le sel interne de (6R-(3(E), 6alpha, 7béta(Z))) 7-(3-(7-(((2-amino 4-thiazolyl) ((diéthoxyphosphinyl (3,4-dihydroxyphényl) méthoxy) imino) acétyl) amino) 2-carboxy 8-oxo 5-thia 1-azabicyclo[4,2,0]oct-2-èn-3-yl) 2-propényl) thiéno (2,3-b)pyridinium,
- le sel interne de (6R-(3(E), 6alpha, 7béta(Z))) 7-(3-(7-(((2-amino 4-thiazolyl) ((3,4-dihydroxyphényl) phosphonométhoxy) imino) acétyl) amino) 2-carboxy 8-oxo 5-thia 1-azabicyclo[4,2,0]oct-2-èn-3-yl) 2-propényl) thiéno(2,3-b)pyridinium,
- le sel interne de (6R-(3(E), 6alpha, 7béta(Z))) 7-(3-(7-(((2-amino 4-thiazolyl) (((éthoxyméthylphosphinyl) (2,5-dichloro 3,4-dihydroxyphényl) méthoxy) imino) acétyl) amino) 2-carboxy 8-oxo 5-thia 1-azabicyclo[4,2,0]oct-2-èn-3-yl) 2-propényl) thiéno(2,3-b)pyridinium (isomère A) et (isomère B),
- le sel interne de (6R-(3(E), 6alpha, 7béta(Z))) 7-(3-(7-(((2-amino 4-thiazolyl) (((hydroxyméthylphosphinyl) (2,5-dichloro 3,4-dihydroxyphényl) méthoxy) imino) acétyl) amino) 2-carboxy 8-oxo 5-thia 1-azabicyclo[4,2,0]oct-2-èn-3-yl) 2-propényl) thiéno(2,3-b)pyridinium,
- le sel interne de (6R-(3(E), 6alpha, 7béta(Z))) 1-(3-(7-(((2-amino 4-thiazolyl) (((éthoxyméthylphosphinyl) (2,5-dichloro 3,4-dihydroxyphényl) méthoxy) imino) acétyl) amino) 2-carboxy 8-oxo 5-thia 1-azabicyclo[4,2,0]oct-2-èn-3-yl) 2-propényl) quinoléinium (isomère A) et (isomère B),
- le sel interne de (6R-(3(E), 6alpha, 7béta(Z))) 1-(3-(7-(((2-amino 4-thiazolyl) (((hydroxyméthylphosphinyl) (2,5-dichloro 3,4-dihydroxyphényl) méthoxy) imino) acétyl) amino) 2-carboxy 8-oxo 5-thia 1-azabicyclo[4,2,0]oct-2-èn-3-yl) 2-propényl) quinoléinium.

L'invention a également pour objet un procédé de préparation des produits de formule (I) telle que définie ci-dessus, caractérisé en ce que l'aldéhyde aromatique de formule (II) :

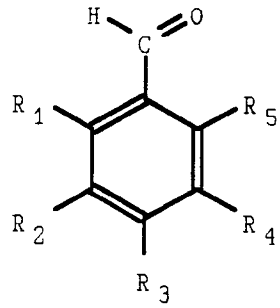

**(II)**

dans laquelle $R_1$, $R_2$, $R_3$, $R_4$ et $R_5$ sont tels que définis ci-dessus, est si nécessaire protégé en ses fonctions réactives et transformé ainsi en aldéhyde aromatique de formule ($II_p$) :

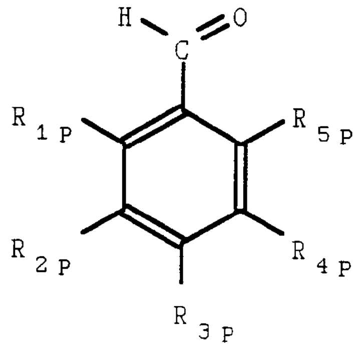

**($II_p$)**

dans laquelle $R_{1p}$, $R_{2p}$, $R_{3p}$, $R_{4p}$ et $R_{5p}$ représentent respectivement les valeurs de $R_1$, $R_2$, $R_3$, $R_4$ et $R_5$ telles que définies précédemment ou une fonction réactive protégée, ledit aldéhyde de formule ($II_p$) est traité par un réactif de formule (III) :

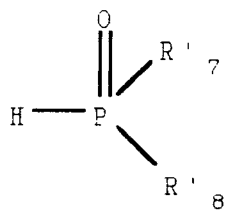

**(III)**

dans laquelle $R'_7$ représente un radical alkyle renfermant de 1 à 4 atomes de carbone, un radical alkyloxy renfermant de 1 à 4 atomes de carbone ou un radical phényle, $R'_8$ représente un radical alkyloxy renfermant de 1 à 4 atomes de carbone ou $R'_7$ et $R'_8$ représentent ensemble un radical alkylènedioxy renfermant de 2 à 8 atomes de carbone,
en présence d'une base, pour obtenir le composé de formule (IV) :

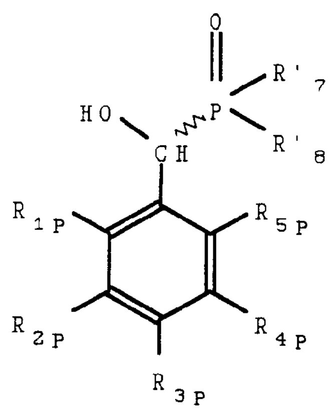

**(IV)**

dans laquelle $R'_7$ et $R'_8$ sont définis comme précédemment et le trait ondulé symbolise un mélange d'isomères que si désiré l'on sépare, et produit de formule (IV) sous forme d'isomère A, d'isomère B ou de mélange, que l'on traite par le N-hydroxy phtalimide, le cas échéant en présence d'un agent activant, pour obtenir le dérivé de formule (V) :

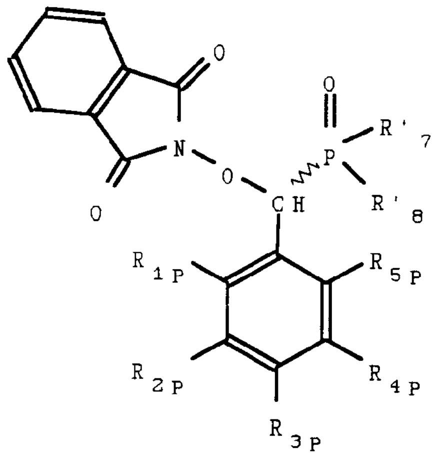

(V)

sous forme d'isomère A, d'isomère B ou de mélange que l'on hydrolyse en hydroxylamine O-substituée de formule (VI) :

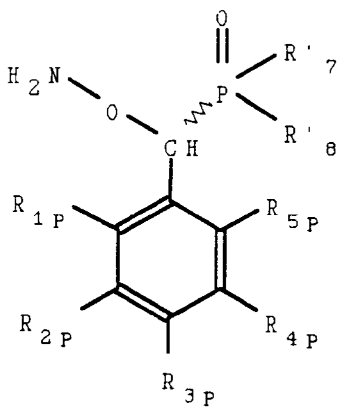

(VI)

sous forme d'isomère A, d'isomère B ou de mélange que l'on condense avec un dérivé de l'acide 2-(2-amino thiazol-4-yl) 2-oxo acétique de formule (VII) :

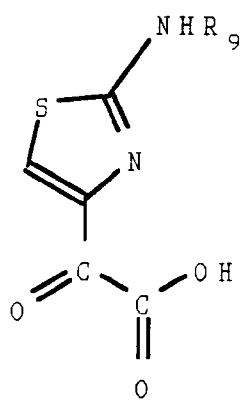

(VII)

dans laquelle $R_9$ représente un atome d'hydrogène ou un groupe protecteur de la fonction amine, pour former le dérivé de l'acide alpha-alkoxyimino acétique "syn" de formule (VIII) :

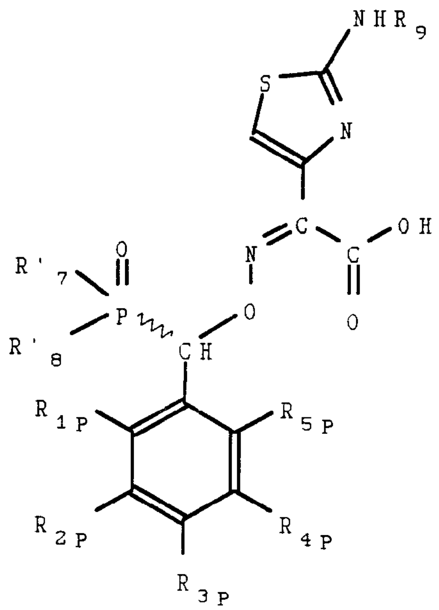

**(VIII)**

sous forme d'isomère A, d'isomère B ou de mélange dont on prépare le cas échéant, un dérivé fonctionnel, produit de formule (VIII) ou dérivé fontionnel que l'on amidifie avec un ester du chlorhydrate de l'acide 7-amino 3-(3-halo 1-propényl) 8-oxo 5-thia 1-azabicyclo[4,2,0]oct-2-èn-2-carboxylique de formule (IX) :

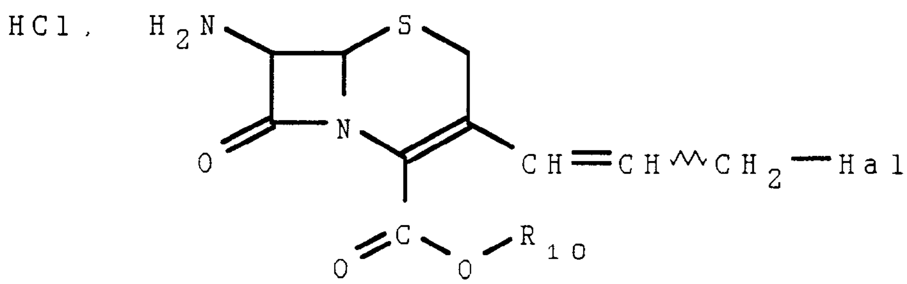

**(IX)**

ou de ses sels, dans laquelle $R_{10}$ représente le reste d'un ester aisément clivable, pour conduire au dérivé de l'acide 7-(N-substitué amido) 3-(3-halo 1-propényl) 8-oxo 5-thia 1-azabicyclo[4,2,0]oct-2-èn-2-carboxylique de formule (X) :

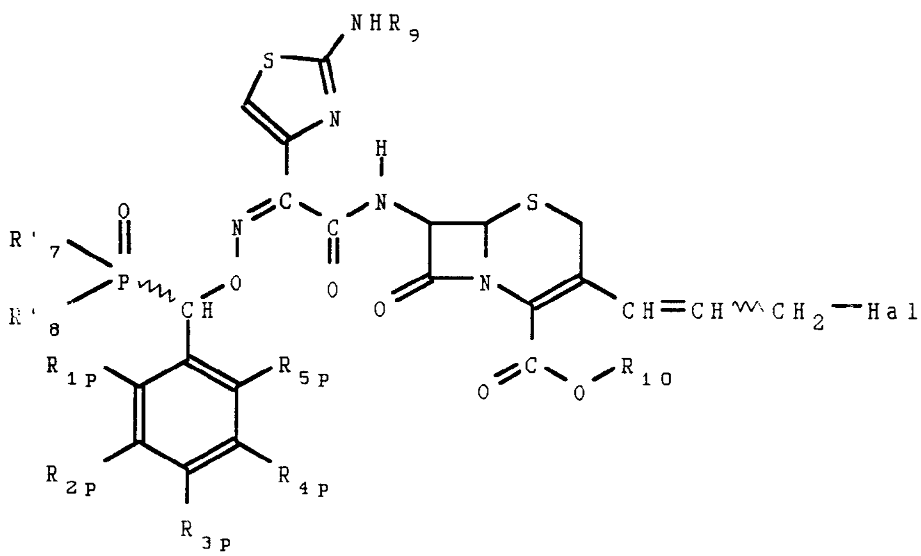

**(X)**

sous forme d'isomère A, d'isomère B ou de mélange que l'on transforme, le cas échéant, en analogue 3-(3-iodo propényle) de formule (XI) :

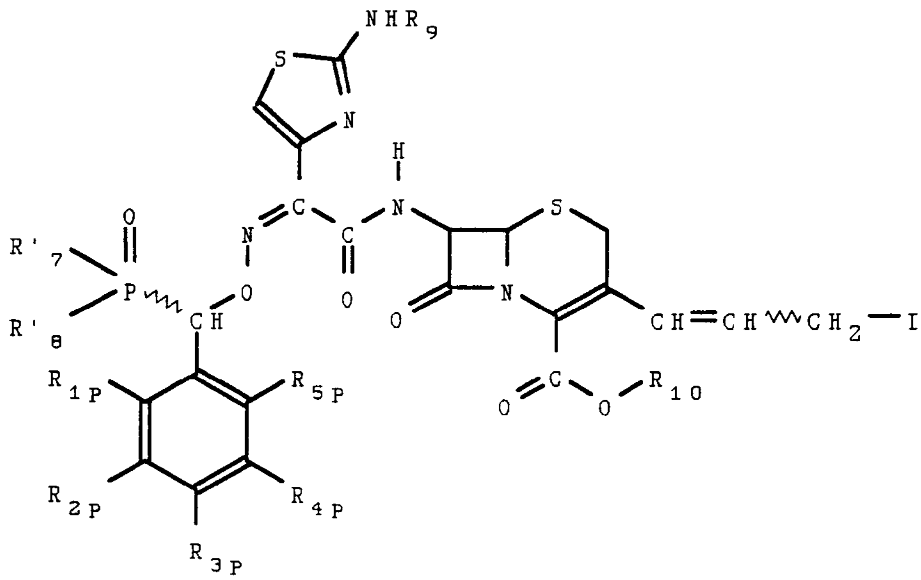

(XI)

sous forme d'isomère A, d'isomère B ou de mélange que l'on traite avec une base de formule $R_6$ pour obtenir le produit de formule (XII) :

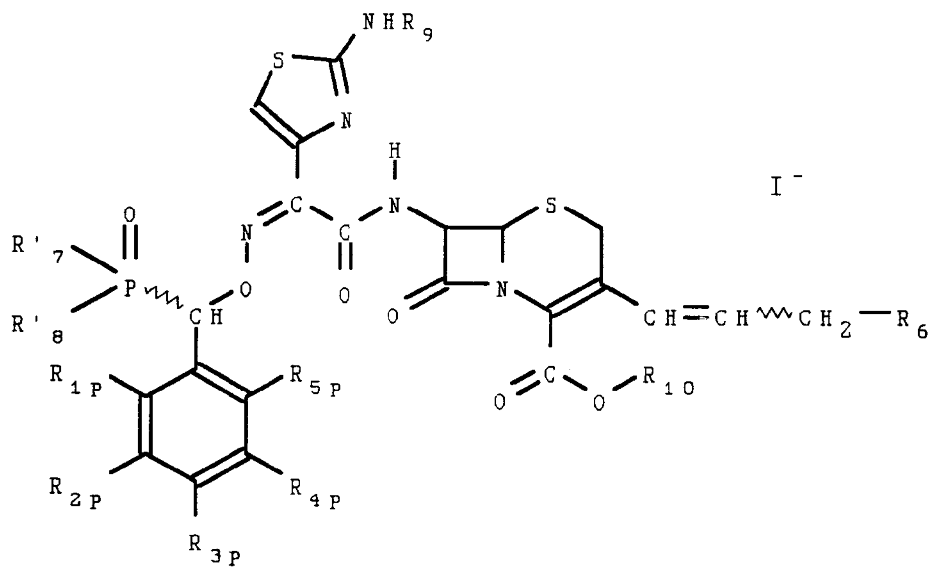

(XII)

sous forme d'isomère A, d'isomère B ou de mélange, produit de formule (XII) à partir duquel si désiré, l'on isole les isomères (E) ou (Z) ou transforme les isomères (Z) en isomère (E), produit de formule (XII) que l'on soumet le cas échéant à une ou plusieurs des réactions suivantes, dans un ordre approprié :

a) coupure par hydrolyse ou par action de la thiourée de tout ou partie des groupements esters ou des groupements de protection du radical amino ou des radicaux hydroxyles,

b) déalkylation d'un ou de deux groupements alkyloxy portés par l'atome de phosphore,

c) estérification ou salification par une base du ou des radicaux carboxyliques et salification par une base du ou des radicaux hydroxy portés par l'atome de phosphore,

d) salification par un acide du radical amino,

e) séparation des produits sous forme de mélange R,S en R et S.

L'invention a aussi pour objet une variante du procédé décrit ci-dessus, caractérisé en ce que l'hydroxylamine

O-substituée de formule (VI) est condensée au produit de formule (XIII) :

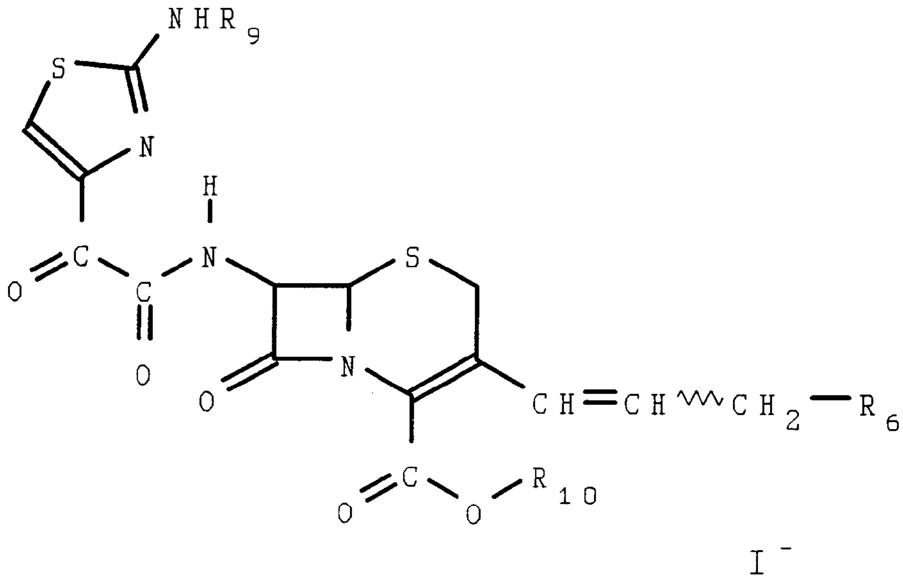

**(XIII)**

pour conduire au produit de formule (XII) telle que définie précédemment.

Les fonctions hydroxy protégées que peuvent représenter $R_{1p}$, $R_{2p}$, $R_{3p}$, $R_{4p}$ et $R_{5p}$, sont choisies parmi les groupes acyloxy tel que par exemple formyloxy, acétoxy, propionyloxy, chloroacétoxy, bromoacétoxy, dichloroacétoxy, trichloroacétoxy, trifluoroacétoxy, méthoxyacétoxy, phénoxyacétoxy, benzoyloxy, benzoylformoxy, p-nitro benzoyloxy. On peut citer également les groupements éthoxycarbonyloxy, méthoxycarbonyloxy, propoxycarbonyloxy, 2,2,2-trichloro éthoxycarbonyloxy, allyloxycarbonyloxy, triméthylsilyléthoxycarbonyloxy, benzyloxycarbonyloxy, tert-butoxycarbonyloxy, 1-cyclopropyl éthoxycarbonyloxy, phtaloyloxy, butyryloxy, isobutyryloxy, valéryloxy, isovaléryloxy, oxalyloxy, succinyloxy et pivaloyloxy, phénylacétoxy, phénylpropionyloxy, mésyloxy, chlorobenzoyloxy, para-nitrobenzoyloxy, para-tert-butyl benzoyloxy, caprylyloxy, acryloyloxy, méthylcarbamoyloxy, phénylcarbamoyloxy, naphtylcarbamoyloxy.

On peut également citer les radicaux phénoxy, 4-chloro phénoxy, tolyloxy ou tert-butyl phénoxy, tolylsulfonyloxy, tétrahydropyrannyloxy, tétrahydrothiopyrannyloxy, méthoxytétrahydropyrannyloxy, trityloxy, benzyloxy, 4-méthoxy benzyloxy, benzhydryloxy, trichloroéthoxy, 1-méthyl 1-méthoxyéthoxy, les radicaux alkoxy alkoxy-méthoxy tel que méthoxy éthoxy méthoxy ou encore les radicaux triméthylsilyléthoxyméthoxy ou triméthylsilyléthoxy.

Deux radicaux hydroxy adjacents peuvent encore être protégés en formant un radical méthylènedioxy, isopropylènedioxy, 1,1-cyclohexyl bis(oxy), diphénylméthylènedioxy, carbonate ou hydroxy borannylbis(oxy).

Les fonctions hydroxy protégées que peuvent représenter $R_{1p}$, $R_{2p}$, $R_{3p}$, $R_{4p}$ et $R_{5p}$, sont choisies de préférence parmi les groupes méthoxyéthoxyméthoxy, propionyloxyméthoxy, acétoxyméthoxy, butoxy, isobutoxy, tert-butoxy, pentoxy, hexyloxy, butyryloxyméthoxy, valéryloxyméthoxy, pivaloyloxyméthoxy, 2-acétoxy éthoxy, 2-propionyloxy éthoxy, 2-butyryloxy éthoxy, 2-iodoéthoxy, 2,2,2-trichloro éthoxy, vinyloxy, allyloxy, éthynyloxy, propynyloxy, benzyloxy, 4-méthoxy benzyloxy, 4-nitro benzyloxy, phényléthoxy, trityloxy, diphénylméthyloxy ou 3,4-diméthoxyphénoxy.

On préfère particulièrement le groupement 2-méthoxy éthoxyméthoxy (MEM-O).

Le reste de groupement ester facilement clivable que représente $R_{10}$ est choisi notamment parmi les groupements butyle, isobutyle, tert-butyle, pentyle, hexyle, méthoxyméthyle, éthoxyméthyle, isopropyloxyméthyle, alpha-méthoxy éthyle, alpha-éthoxy éthyle, méthylthiométhyle, éthylthiométhyle, isopropylthiométhyle, pivaloyloxyméthyle, acétoxyméthyle, propionyloxyméthyle, butyryloxyméthyle, isobutyryloxyméthyle, valéryloxyméthyle, isovaléryloxyméthyle, tert-butylcarbonyloxyméthyle, hexadécanoyloxyméthyle, pivaloyloxyméthyl, propionyloxyéthyle, isovaléryloxyéthyle, 1-acétoxy éthyle, 2-acétoxy éthyle, 1-propionyloxy éthyle, 2-propionyloxy éthyle, 1-butyryloxy éthyle, 2-butyryloxy éthyle, 1-(tert-butylcarbonyloxy) éthyle, 1-acétoxy propyle, 1-hexadécanoyloxy éthyle, 1-propionyloxy propyle, 1-méthoxycarbonyloxy éthyle, méthoxycarbonyloxyméthyle, 1-acétoxy butyle, 1-acétoxy hexyle, 1-acétoxy heptyle, phtalidyle, 5,6-diméthoxy phtalidyle, tert-butylcarbonylméthyle, vinyle, allyle, 2-chloro allyle, éthynyle, propynyle, méthoxycarbonylméthyle, benzyle, 4-méthoxy benzyle, 4-nitro benzyle, phénéthyle, trityle, diphényl méthyle, phényle, 4-chloro phényle, tolyle, tert-butyl phényle, 3,4-diméthoxy phényle, méthoxyéthoxyméthyle, diméthylaminoéthyle, cyanométhyle, tert-butoxycarbonylméthyle, 2,2-éthylènedioxy éthyle, cyanoéthyle, 2,2-diméthoxy éthyle, 2-chloro éthoxyméthyle, (2-hydroxy éthoxy) éthyle, 2,3-époxy propyle, 3-diméthylamino 2-hydroxy propyle, 2-hydroxy éthyle, 2-méthylaminoéthoxyméthyle, (2-amino éthoxy) méthyle, 3-méthoxy 2,4-thiadiazol-5-yle, tétrahydropyrann-2-yle, 1-méthoxy 1-méthyl éthyle, 2-hydroxy 1-méthyl éthyle, isopropyle, carbamoylméthyle, chlorométhyle, 2-chloro éthyle, 2,2,2-trichloro éthyle, 2-iodo éthyle, acétyle, méthyle, 2-méthylthio éthyle, thiocyanatométhyle, 2-chloro 1-acétoxy éthyle, 2-bromo 1-acétoxy éthyle, 2-fluoro 1-acétoxy éthyle, 2-méthoxy 1-acétoxy éthyle, 2-méthyl 1-acétoxy propyle, 1-méthyl 1-acétoxy éthyle, 1-(méthoxyacétoxy) éthyle, 1-acétyl carbonyloxyéthyle, 1-hydroxy acétoxyéthyle, 1-(2-thiényl) carbonyloxyéthyle, 1-(2-furyl) carbonyloxyéthyle, 1-(5-nitro 2-furyl) carbonyloxyéthyle, 1-(2-pyrrolyl) carbonyloxyéthyle, 1-(propionyloxycarbonyloxy) éthyle, 1-(propoxycarbonyloxy) éthyle, 1-(isopropoxycarbonyloxy) éthyle, 1-(méthoxyéthoxycarbonyloxy) éthyle, 1-(allyloxycarbonyloxy) éthyle, isopropoxycarbonyl méthyle, 1-[(2,3-époxy propyl) oxycarbonyloxy] éthyle, 1-[(2-furyl) méthoxycarbonyloxy] éthyle, 1-[(2-fluoro éthoxy) carbonyloxy] éthyle, 1-(méthoxycarbonyloxy) propyle, 1-(méthoxycarbonyloxy) 1-méthyl éthyle, (méthoxycarbonyloxy) chlorométhyle, 1-(méthoxycarbonyloxy) 2-chloro éthyle, 1-(méthoxycarbonyloxy) 2-méthoxy éthyle, 1-(méthoxycarbonyloxy) allyle ou 5-méthyl 2-oxo 1,3-dioxol-4-yle.

Le radical diphénylméthyle est plus particulièrement préféré.

Le groupement protecteur du radical amino que peut représenter $R_9$ peut être par exemple un groupe carbamoyle, méthyl carbamoyle, phénylcarbamoyle, naphtylcarbamoyle, ainsi que les thiocarbamoyles correspondants, un radical alkyle de 1 à 6 atomes de carbone substitué ou non substitué tel que, préférentiellement, trichloroéthyle, tert-butyle ou tert-amyle, un radical aralkyle tel que benzyle, 4-méthoxy benzyle, phénéthyle, trityle, 3,4-diméthoxy benzyle ou benzhydryle, un radical acyle aliphatique, aromatique ou hétérocyclique substitué ou non, tel que par exemple formyle, acétyle, propionyle, butyryle, isobutyryle, valéryle, isovaléryle, chloroacétyle, dichloroacétyle, trichloroacétyle, bromoacétyle, trifluoroacétyle benzoyle, toluolyle, naphtoyle, chlorobenzoyle, para-nitro benzoyle, para-tert-butyl benzoyle, phénoxyacétyle, caprylyle, décanoyle, acryloyle, phtaloyle, mésyle, phénylacétyle, phénylpropionyle, oxalyle, succinyle, pivaloyle, un radical alcoxycarbonyle ou cycloalcoxycarbonyle inférieur tel que par exemple, méthoxycarbonyle, éthoxycarbonyle, propoxycarbonyle, 1-cyclopropyléthoxycarbonyle, isopropoxycarbonyle, butoxycarbonyle, tert-butoxycarbonyle, pentoxycarbonyle, hexyloxycarbonyle, trichloroéthoxy carbonyle, un groupe aralcoxycarbonyle, tel que benzyloxycarbonyle.

On préfère le groupement trityle.

La liste ci-dessus n'est pas limitative, il est évident que d'autres groupements protecteurs des amines, groupements connus en particulier dans la chimie des peptides, peuvent également être utilisés.

La base en présence de laquelle on fait agir le réactif de formule (III) peut être une amine, notamment une amine tertiaire telle que la triéthylamine ou la pyridine ou encore un hydroxyde faiblement basique, notamment l'hydroxyde d'aluminium ou de baryum, ou encore un carbonate ou bicarbonate alcalin.

L'agent activant en présence duquel on fait agir le N-hydroxyphtalimide peut être un agent de transfert de phase. De tels agents sont connus de l'homme du métier.

L'hydrolyse du phtalimide de formule (V) est effectuée par action de l'hydrazine, de préférence sous forme d'hydrate.

Le dérivé fonctionnel de l'acide de formule (VIII) peut être par exemple un halogénure, un anhydride symétrique ou mixte, l'amide, l'azide ou un ester activé.

Comme exemple d'anhydride mixte on peut citer par exemple celui formé avec le chloroformiate d'isobutyle et celui formé avec le chlorure de pivaloyle et les anhydrides mixtes carboxylique-sulfonique formés par exemple avec le chlorure de para-toluène sulfonyle.

Comme exemple d'ester activé, on peut mentionner l'ester formé avec le 2,4-dinitrophénol et celui formé avec l'hydroxybenzothiazole.

Comme exemple d'halogénure, on peut citer le chlorure ou le bromure.

L'anhydride peut être formé in situ par action de carbodiimide N,N'-disubstitué, par exemple le N,N-dicyclohexylcarbodiimide.

La réaction d'acylation est conduite de préférence dans un solvant organique tel que le chlorure de méthylène. On peut cependant utiliser d'autres solvants tels que le tétra-hydrofuranne, le chloroforme ou le diméthylformamide.

Lorsqu'on utilise un halogénure d'acide et de manière générale lorsqu'une molécule d'acide est libérée au cours de la réaction, on réalise la réaction de préférence en présence d'une base telle que la soude, la potasse, les carbonates et carbonates acides de sodium ou de potassium, l'acétate de sodium, la triéthylamine, la N,N-diisopropyl éthyl amine, la pyridine, la morpholine ou la N-méthylmorpholine.

La température de réaction est en général inférieure ou égale à la température ambiante.

On peut également faire agir directement un produit de formule (VIII) avec un produit de formule (IX) en présence d'un carbodiimide telle que le diisopropylcarbodiimide ou le 1-(3-diméthylamino propyl) 3-éthyl carbodiimide (EDC). Un exemple d'une telle préparation est donnée plus loin dans la partie expérimentale.

L'action des réactifs capable d'introduire le radical $R_6$ et conduire au produit de formule (XII) est effectuée dans les conditions suivantes :

Lorsque Hal représente par exemple un atome de chlore, on peut effectuer in situ ou séparément une substitution de l'atome de chlore par un atome d'iode en présence d'iodure de sodium puis ajoute ensuite le réactif désiré, en présence ou non d'un solvant organique tel que le dichlorométhane, l'acétonitrile, le tétrahydrofuranne, l'acétone ou la méthyléthylcétone.

On peut également faire agir directement sur le produit de formule (X) ou (XI), le réactif de formule $R_6$ désiré en présence de tétrafluoroborate d'argent.

L'isomérie des produits de formule (XII) peut être différente de celle des produits de formule (X) ou (XI). Dans le

cas où l'on isole l'isomère Z, on peut transformer cet isomère en isomère E selon les méthodes usuelles, notamment par action de l'iode.

Selon les valeurs de $R_9$, $R_{10}$, $R_{1p}$, $R_{2p}$, $R_{3p}$, $R_{4p}$ et $R_{5p}$, l'action sur le produit de formule (XII) d'un ou plusieurs agents d'hydrolyse, d'hydrogénolyse ou de la thiourée a pour but d'éliminer le radical $R_9$ lorsque celui-ci représente un groupement protecteur du radical amino, de transformer les radicaux $R_{1p}$, $R_{2p}$, $R_{3p}$, $R_{4p}$ et $R_{5p}$ respectivement en radicaux $R_1$, $R_2$, $R_3$, $R_4$ et $R_5$ lorsque ceux-ci portent un groupement protecteur des radicaux hydroxyle et/ou d'éliminer le radical $R_{10}$ lorsque celui-ci représente, parmi les groupements esters facilement clivables l'un de ceux que l'on désire éliminer.

Cependant, il est bien entendu possible d'éliminer $R_9$ et de transformer les radicaux $R_{1p}$, $R_{2p}$, $R_{3p}$, $R_{4p}$ et $R_{5p}$ respectivement en radicaux $R_1$, $R_2$, $R_3$, $R_4$ et $R_5$ lorsque ceux-ci portent un groupement protecteur des radicaux hydroxyle sans toucher au substituant $R_{10}$ lorsque celui-ci doit être conservé. La nature des réactifs à mettre en jeu dans un tel cas est bien connu de l'homme du métier. On trouvera par exemple une description des différentes méthodes d'élimination des différents groupements protecteurs dans le brevet français B.F. 2.499.995. Des exemples de telles réactions sont donnés plus loin dans la partie expérimentale.

Etant donné la nature des groupements protecteurs préférés que l'on utilise : trityle pour $R_9$, 2-méthoxy éthoxy méthyle pour protéger les fonctions hydroxy et 4-méthoxy benzyle pour $R_{10}$, on utilise de préférence l'acide trifluoroacétique sans solvant ou dans un solvant tel que l'anisole ou un mélange de solvants tels que anisole/chlorure de méthylène. On obtient alors un sel avec l'acide trifluoroacétique. On peut revenir à la base libre par action d'une base telle qu 'un carbonate ou la triéthylamine.

La déalkylation éventuelle, partielle ou totale du ou des groupements alkyloxy portés par l'atome de phosphore est effectuée de préférence dans des conditions douces. On opère de préférence par action d'un halogénure de trialkyle silyle, notamment le bromure ou l'iodure de triméthylsilyle, l'intermédiaire silylé obtenu étant hydrolysé par action d'un alcool, par exemple le méthanol ou l'éthanol. On peut encore opérer par action du bromure de lithium. La déalkylation est de préférence effectuée au dernier stade, c'est-à-dire après élimination des divers groupements protecteurs. Le cas échéant, ceci peut bien entendu ne pas être une obligation. Le choix de l'ordre des réactions est à la portée de l'homme du métier.

La salification des produits peut être effectuée selon les méthodes usuelles ; elle peut par exemple être obtenue par action sur un produit sous forme acide ou sur un solvat, par exemple le solvat éthanolique, ou un hydrate de cet acide, d'une base minérale telle que l'hydroxyde de sodium ou de potassium, le carbonate ou le carbonate acide de sodium ou de potassium. On peut également utiliser les sels d'acides minéraux tels que le phosphate trisodique. On peut également faire appel à des sels d'acides organiques tels que par exemple, les sels de sodium d'acides carboxyliques aliphatiques, linéaires ou ramifiés, saturés ou insaturés de 1 à 18 et de préférence de 2 à 10 atomes de carbone. Les chaînes aliphatiques de ces acides peuvent être interrompues par un ou plusieurs hétéroatomes tels que l'oxygène ou le soufre ou substituées par des radicaux aryle tels que phényle, thiényle ou furyle, par un ou plusieurs radicaux hydroxyles ou par un ou plusieurs atomes d'halogène tels que fluor, chlore ou brome, préférentiellement chlore, par un ou plusieurs radicaux carboxyliques ou alkoxycarbonyles inférieurs, de préférence méthoxycarbonyle, éthoxycarbonyle ou propyloxycarbonyle, par un ou plusieurs radicaux aryloxy, de préférence phénoxy.

De plus, on peut utiliser comme acides organiques, des acides aromatiques suffisamment solubles comme, par exemple, des acides benzoïques substitués, de préférence par des radicaux alkyles inférieurs.

Comme exemples de tels acides organiques, on peut mentionner les acides formique, acétique, acrylique, butyrique, adipique, isobutyrique, n-caproïque, isocaproïque, chloropropionique, crotonique, phényl acétique, (2-thiényl) acétique, (3-thiényl) acétique, (4-éthyl phényl) acétique, glutarique, l'ester monoéthylique de l'acide adipique, les acides hexanoïque, heptanoïque, décanoïque, oléïque, stéarique, palmitique, 3-hydroxy propionique, 3-méthoxy propionique, 3-méthyl thiobutyrique, 4-chloro butyrique, 4-phényl butyrique, 3-phénoxy butyrique, 4-éthyl benzoïque, 1-propyl benzoïque.

On utilise cependant de préférence comme sels de sodium, l'acétate de sodium, le 2-éthyl hexanoate de sodium ou le diéthyl acétate de sodium.

La salification peut également être obtenue par action d'une base organique comme la triéthylamine, la diéthylamine, la triméthylamine, la propylamine, la N,N-diméthyl éthanolamine, le tris[(hydroxyméthyl) amino] méthane, la méthylamine, l'éthanolamine, la pyridine, la picoline, la dicyclohexyl amine, la morpholine et la benzylamine.

Elle peut également être obtenue par action de l'arginine, de la lysine, de la procaïne, de l'histidine, de la N-méthyl glucamine.

Cette salification est réalisée de préférence dans un solvant ou un mélange de solvants tels que l'eau, l'éther éthylique, le méthanol, l'éthanol ou l'acétone.

Les sels sont obtenus sous forme amorphe ou cristallisée selon les conditions réactionnelles employées.

Les sels cristallisés sont préparés de préférence en faisant réagir les acides libres avec l'un des sels des acides carboxyliques aliphatiques mentionnés ci-dessus.

La salification des produits par les acides minéraux ou organiques est effectuée dans les conditions usuelles.

L'estérification éventuelle des produits est effectuée dans les conditions classiques. On opère en général en faisant

réagir l'acide de formule (I) ou un dérivé fonctionnel avec un dérivé de formule :

Z-Re

dans laquelle Z représente un radical hydroxyle ou un atome d'halogène tel que le chlore, le brome, l'iode et Re désigne le groupement ester à introduire, groupement dont une liste non exhaustive figure ci-dessus. Dans certains cas, il peut être avantageux d'effectuer une estérification sur un produit dont l'amine et/ou les groupements réactionnels présents sur l'oxyimino sont bloqués avant d'enlever le groupement protecteur de l'amine et du groupement réactionnel présents sur l'oxyimino.

Les produits de formule (I) comportent plusieurs carbones asymétriques. Dans le noyau cephème, qui comporte deux carbones asymétriques, les deux carbones sont dans la configuration R. Par ailleurs, le radical présent sur la fonction oxyimino comporte également un carbone asymétrique qui peut être sous forme R ou S ou sous forme d'un mélange R + S. La séparation des deux diastéréoisomères peut être effectuée par les moyens connus de l'homme du métier, par exemple par chromatographie.

Les produits de formule générale (I) possèdent une très bonne activité antibiotique sur les bactéries gram $^{\oplus}$ telles que les staphylocoques, les streptocoques et notamment sur les staphylocoques pénicillino-résistants. Leur efficacité sur les entérobactéries productrices de β-lactamases chromosomales ou plasmidiques, est particulièrement remarquable.

Ces propriétés rendent aptes lesdits produits ainsi que leurs sels d'acides pharmaceutiquement acceptables à être utilisés comme médicaments dans le traitement des infections à germes sensibles et notamment dans celui des staphylococcies, telles que septicémies à staphylocoques, staphylococcies malignes de la face ou cutanée, pyodermites, plaies septiques ou suppurantes, anthrax, phlegmons, érésipèles, staphylococcies aiguës primitives ou post grippales, bronchopneumonies, suppurations pulmonaires.

Ces produits peuvent également être utilisés comme médicaments dans le traitement des colibacilloses et infections associées, dans les infections à proteus, à klebsiella et à salmonella et dans d'autres affections provoquées par des bactéries à gram $^{\ominus}$.

La présente invention a donc également pour objet, à titre de médicaments et notamment de médicaments antibiotiques, les produits de formule (I) tels que définis ci-dessus ainsi que leurs sels d'acides pharmaceutiquement acceptables.

L'invention a particulièrement pour objet à titre de médicaments les produits de formule (I) telle que décrite ci-dessus dans laquelle $R_6$ est choisi parmi les radicaux quinoléinium, isoquinoléinium, 4-(méthylthio) pyridinium, thiéno-[2,3-b] pyridinium, 1-méthyl pyrrolidinium, N-méthyl N-éthyl N-(2-amino 2-oxoéthyl) aminium, imidazo (1,2-a) pyridinium et 6,7-dihydro 5H-pyrindinium, ceux dans laquelle $R_3$ et $R_4$ représentent chacun un radical hydroxy, ceux dans laquelle $R_2$ et $R_5$ représentent chacun un atome de chlore ou de fluor, ceux dans laquelle $R_1$ et $R_2$ représentent chacun un atome de fluor et ceux dans laquelle $R_2$ représente un radical méthoxy et l'un de $R_1$ ou $R_5$ représente un atome de chlore.

L'invention a plus particulièrement pour objet, à titre de médicaments et notamment de médicaments antibiotiques, les produits dont les noms suivent :

- le sel interne de (6R-(3-(E) 6alpha, 7béta-(Z))) 1-(3-(7-(((2-amino 4-thiazolyl) ((éthoxyméthylphosphinyl (3,4-dihydroxyphényl) méthoxy) imino) acétyl) amino) 2-carboxy 8-oxo 5-thia 1-azabicyclo[4,2,0]oct-2-èn-3-yl) 2-propényl) quinoléinium,
- le sel interne de (6R-(3-(E) 6alpha, 7béta-(Z))) 7-(3-(7-(((2-amino 4-thiazolyl) ((éthoxyméthylphosphinyl (3,4-dihydroxyphényl) méthoxy) imino) acétyl) amino) 2-carboxy 8-oxo 5-thia 1-azabicyclo[4,2,0]oct-2-èn-3-yl) 2-propényl) thiéno(2,3-b)pyridinium,
- le sel interne de (6R-(3-(E) 6alpha, 7béta-(Z))) 7-(3-(7-(((2-amino 4-thiazolyl) ((hydroxyméthylphosphinyl (3,4-dihydroxyphényl) méthoxy) imino) acétyl) amino) 2-carboxy 8-oxo 5-thia 1-azabicyclo[4,2,0]oct-2-èn-3-yl) 2-propényl) thiéno(2,3-b)pyridinium,
- le sel interne de (6R-(3-(E) 6alpha, 7béta-(Z))) 7-(3-(7-(((2-amino 4-thiazolyl) (((2,5-dichloro 3,4-dihydroxyphényl) (diéthoxyphosphinyl) méthoxy) imino) acétyl) amino) 2-carboxy 8-oxo 5-thia 1-azabicyclo[4,2,0]oct-2-èn-3-yl) 2-propényl) thiéno(2,3-b)pyridinium,
- le sel interne de (6R-(3-(E) 6alpha, 7béta-(Z))) 7-(3-(7-(((2-amino 4-thiazolyl) (((2,5-dichloro 3,4-dihydroxyphényl) phosphonométhoxy) imino) acétyl) amino) 2-carboxy 8-oxo 5-thia 1-azabicyclo[4,2,0]oct-2-èn-3-yl) 2-propényl) thiéno(2,3-b)pyridinium,
- le sel interne de (6R-(3(E), 6alpha, 7béta(Z))) 7-(3-(7-(((2-amino 4-thiazolyl) ((diéthoxyphosphinyl (3,4-dihydroxyphényl) méthoxy) imino) acétyl) amino) 2-carboxy 8-oxo 5-thia 1-azabicyclo[4,2,0]oct-2-èn-3-yl) 2-propényl) thiéno

(2,3-b)pyridinium,

- le sel interne de (6R-(3(E), 6alpha, 7béta(Z))) 7-(3-(7-(((2-amino 4-thiazolyl) ((3,4-dihydroxyphényl) phosphonométhoxy) imino) acétyl) amino) 2-carboxy 8-oxo 5-thia 1-azabicyclo[4,2,0]oct-2-èn-3-yl) 2-propényl) thiéno(2,3-b)pyridinium,

- le sel interne de (6R-(3(E), 6alpha, 7béta(Z))) 7-(3-(7-(((2-amino 4-thiazolyl) (((éthoxyméthylphosphinyl) (2,5-dichloro 3,4-dihydroxyphényl) méthoxy) imino) acétyl) amino) 2-carboxy 8-oxo 5-thia 1-azabicyclo[4,2,0]oct-2-èn-3-yl) 2-propényl) thiéno(2,3-b)pyridinium (isomère A) et (isomère B),

- le sel interne de (6R-(3(E), 6alpha, 7béta(Z))) 7-(3-(7-(((2-amino 4-thiazolyl) (((hydroxyméthylphosphinyl) (2,5-dichloro 3,4-dihydroxyphényl) méthoxy) imino) acétyl) amino) 2-carboxy 8-oxo 5-thia 1-azabicyclo[4,2,0]oct-2-èn-3-yl) 2-propényl) thiéno(2,3-b)pyridinium,

- le sel interne de (6R-(3(E), 6alpha, 7béta(Z))) 1-(3-(7-(((2-amino 4-thiazolyl) (((éthoxyméthylphosphinyl) (2,5-dichloro 3,4-dihydroxyphényl) méthoxy) imino) acétyl) amino) 2-carboxy 8-oxo 5-thia 1-azabicyclo[4,2,0]oct-2-èn-3-yl) 2-propényl) quinoléinium (isomère A) et (isomère B),

- le sel interne de (6R-(3(E), 6alpha, 7béta(Z))) 1-(3-(7-(((2-amino 4-thiazolyl) (((hydroxyméthylphosphinyl) (2,5-dichloro 3,4-dihydroxyphényl) méthoxy) imino) acétyl) amino) 2-carboxy 8-oxo 5-thia 1-azabicyclo[4,2,0]oct-2-èn-3-yl) 2-propényl) quinoléinium,

sous leurs formes isomériques (R) ou (S) ou de mélanges (R+S), ainsi que leurs sels pharmaceutiquement acceptables.

L'invention s'étend aux compositions pharmaceutiques renfermant, comme principe actif, au moins un des médicaments définis ci-dessus.

Ces compositions peuvent être administrées par voie buccale, rectale, parentérale, notamment intramusculaire ou par voie locale en application topique sur la peau et les muqueuses.

Les produits de formule (I) et notamment ceux dans laquelle A représente un ester clivable peuvent être administrés par voie orale.

Les compositions selon l'invention peuvent être solides ou liquides et se présenter sous les formes pharmaceutiques couramment utilisées en médecine humaine comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent notamment se présenter sous forme d'une poudre destinée à être dissoute extemporanément dans un véhicule approprié, par exemple, de l'eau stérile apyrogène.

La dose administrée est variable selon l'affection traitée, le sujet en cause, la voie d'administration et le produit considéré. Elle peut être, par exemple, comprise entre 0,250 g et 4 g par jour, par voie orale chez l'homme, avec le produit décrit à l'exemple 1 ou encore comprise entre 0,500 g et 1 g trois fois par jour par voie intramusculaire.

Les produits de formule (I) peuvent également être utilisés comme désinfectants des instruments chirurgicaux.

L'invention a enfin pour objet, à titre de produits industriels nouveaux et notamment à titre de produits intermédiaires nécessaires à la préparation des produits de formule (I), les produits de formules (IV), (V), (VI) (VIII), (X), (XI) et (XII) telles que définies précédemment.

Les produits de formule (II) sont connus de manière générale et pour la plupart commercialisés ; d'autres peuvent être préparés à partir de produits commercialisés, selon les méthodes décrites dans la demande européenne 0551034. Pour la préparation des produits de formule (II), on peut aussi utiliser les méthodes décrites dans la littérature, notamment la réduction dite de Rosemund, la réduction des acides benzoïques, ou la formylation des cycles aromatiques comme par exemple la réaction de Vilsmeier-Haack, la réaction de Gatterman-Koch, la réaction de Reimer-Tiemann ou la réaction avec le fluorure de formyle (J. Am. Chem. Soc. 82, 2380 (1960)).

Les produits de formules (VII) et (IX) sont également connus dans la littérature, notamment dans les demandes de brevets belge BE864828 et européenne EP0333154.

La préparation des produits de formule (XIII) est décrite dans la demande européenne 0551034.

Le réactif de formule (III) peut être préparé comme décrit, par exemple dans Derwent 81.82015 D/45 Série C.

Les exemples suivants illustrent l'invention sans toutefois la limiter.

**<u>EXEMPLE 1</u> : Sel interne de (6R(3-(E), 6alpha, 7béta(Z))) 1-(3-(7-(((2-amino 4-thiazolyl) (éthoxyméthylphosphinyl (3,4-dihydroxyphényl) méthoxy) imino) amino) 2-carboxy 8-oxo 5-thia 1-azabicyclo(4.2.0)oct-2-èn-3-yl) 2-propényl) quinoléinium**

<u>STADE A</u> : (hydroxy (3,4-bis((2-méthoxyéthoxy) méthoxy) phényl) méthyl) méthylphosphinate d'éthyle

On agite pendant 24 heures à 75°C une solution comprenant 9 g de 3,4-bis-[(méthoxyéthoxy) méthoxy] benzaldéhyde, 3,26 g de réactif méthyl-éthyl phosphite préparé ci-dessous et 7,97 $cm^3$ de triéthylamine. On évapore la triéthylamine sous pression réduite, purifie le résidu sur silice en éluant à l'acétate d'éthyle puis avec le système acétate d'éthyle/ éthanol 95-5, 90-10 puis 85-15.

On obtient 6,85 g de produit attendu.

Rf = 0,2 (éluant ACOEt/EtOH 95-5).

Préparation du réactif méthyl-éthyl phosphite.

On refroidit à 0°C une solution de 25 g du dichlorométhylphosphine dans 150 $cm^3$ d'éther puis ajoute goutte à goutte en 45 minutes 30 $cm^3$ de triéthylamine et 30 $cm^3$ d'éthanol en solution dans 40 $cm^3$ d'éther. On agite 19 heures à température ambiante, élimine par filtration le chlorhydrate de triéthylamine, rince à l'éther et évapore les solvants. Après distillation du résidu sous pression réduite (13 mbars) on recueille 12,9 g de réactif entre 56° et 58°C.

rf = 0,2 (ACOEt - EtOH 95-5).

<u>STADE B</u> : ((3,4-bis((2-méthoxyéthoxy) méthoxy) phényl) ((1,3-dihydro 1,3-dioxo-2H-isoindol 2-yl) oxy) méthyl) méthylphosphinate d'éthyle

A 6,59 g de l'alcool obtenu au stade A en solution dans 400 $cm^3$ de tétrahydrofuranne, on ajoute 8,17 g de triphényl phosphine et 2,8 g de N-hydroxyphtalimide puis refroidit le milieu réactionnel à 0°/-5°C.

On ajoute 5,43 g de diéthyl azadicarboxylate puis agite 3 heures en laissant revenir à température amibante. On ajoute 150 $cm^3$ d'eau, extrait au dichlorométhane, sèche et évapore le solvant sous pression réduite.

On purifie par chromatographie sur silice (éluant acétate d'éthyle puis acétate d'éthyle/éthanol 95-5).

On obtient 1,32 g de produit attendu.

rf = 0,35 (ACOEt/EtOH 95-5).

Spectre RMN (300 MHz $CDCl_3$ ppm)

| | |
|---|---|
| 1,34 | $P-O-CH_2-\underline{CH_3}$ |
| 1,68-1,77 | $-P-CH_3$ |
| 3,55-3,87 et 5,29 | O-MEM |
| 4,0 à 4,3 | $P-O-\underline{CH_2}-CH3$ |
| 5,55-5,67 | CH ~ P- |
| 7,17-7,53 | phényl |
| 7,72 | phtalimide |

<u>STADE C</u> : aminooxy (3,4-bis((2-méthoxyéthoxy) méthoxy) phényl) méthyl) méthylphosphinate d'éthyle

On ajoute 1,34 g d'hydrate d'hydrazine à 4,62 g de produit obtenu au stade B en solution dans 120 $cm^3$ d'éthanol puis agite 1 heure à température ambiante.

On filtre, évapore le solvant sous pression réduite et obtient 3,16 g de produit attendu.

rf = 0,1 (ACOEt/EtOH 95-5)

Spectre RMN ($CDCl_3$ 250 MHz ppm)

| | |
|---|---|
| 1,28 | $-P-O-CH_2-\underline{CH_3}$ |
| 1,43-1,44 | $-P-CH_3$ |
| 3,35-3,38-3,56-3,86 et 5,32 | OMEM |
| 3,8 à 4,3 | $-P-O-\underline{CH_2}-CH_3$ |

| | |
|---|---|
| 4,84 | -CH~ P- |
| 6,9 à 7,25 | aromatiques. |

STADE D : (2) acide α-((éthoxyméthylphosphinyl) (3,4-((2-méthoxyéthoxy) méthoxy) phényl) méthoxy)(2-((triphénylméthyl) amino) 4-thiazole acétique

On agite 3 heures à température ambiante 2,95 g du produit obtenu au stade C en solution dans 60 $cm^3$ de méthanol et 2,94 g d'acide oxo-[2-[(triphénylméthyl) amino] thiazol 4-yl] acétique (décrit dans la demande de brevet belge n° 864828. On évapore le solvant sous pression réduite, purifie par chromatographie sur silice (éluant dichlorométhane/méthanol 90-10 puis 80-20).
On obtient 4,43 g de produit attendu.
rf = 0,55 ($CH_2Cl_2$/MeOH 8-2)
Spectre RMN ($CDCl_3$ ppm)

| | |
|---|---|
| 1,03 | -P-O-$CH_2$-<u>$CH_3$</u> |
| 1,20-1,57 | -P-$CH_3$ |
| 3,27-3,34-3,51-3,8-4,12 | -OMEM |
| et 5,2 à 5,52 | |
| 3,51-3,8-4,12 | -P-O-<u>$CH_2$</u>-$CH_3$ |
| 5,2 à 5,52 | -CH~ P- |
| 6,64 | $H_5$ du thiazole |
| 6,9 à 7,23 | aromatiques. |

STADE E : [6R(3(E), 6alpha, 7béta(Z))] 3-(3-chloro 1-propényl) 7-(((((éthoxyméthylphosphinyl) (3,4-bis((2-méthoxyéthoxy) méthoxy) phényl) méthoxy) imino) (2-((triphénylméthyl) amino) 4-thiazolyl) acétyl) amino) 8-oxo 5-thia 1-azabicyclo(4.2.0)oct-2-ène-2-carboxylate de (4-méthoxyphényl) méthyle

A une solution de 4,3 g d'acide obtenu au stade D dans 200 $cm^3$ de dichlorométhylène, on ajoute 2,24 g de chlorhydrate de 7β-amino 3-[(Z) 3-chloro 1-propényl] 8-oxo 5-thia 1-azabicyclo[4.2.0]oct-2-ène 2-carboxylate de 4-méthoxy benzyle (décrit dans la demande de brevet européen n°-0333154) puis refroidit le milieu réactionnel à -5°C. On ajoute 1,29 g de diméthylamino propyl-éthyl-carbodiimide (EDC) et poursuit l'agitation à -5°/0°C pendant 3 heures. On ajoute 50 $cm^3$ de tampon phosphate pH 7, extrait au dichlorométhane puis à l'acétate d'éthyle, sèche et évapore le solvant sous pression réduite. On obtient après purification sur silice (éluant dichlorométhane-acétone (8-2)) 3,92 g de produit attendu.
rf = 0,35 ($CH_2Cl_2$-acétone 7-3).

STADE F : [6R(3(E), 6alpha, 7béta(Z))] 3-(3-iodo 1-propényl) 7-(((((éthoxyméthylphosphinyl) (3,4-bis((2-méthoxyéthoxy) méthoxy) phényl) méthoxy) imino) (2-((triphénylméthyl) amino) 4-thiazolyl) acétyl) amino) 8-oxo 5-thia 1-azabicyclo(4.2.0) oct-2-ène-2-carboxylate de (4-méthoxyphényl) méthyle

On ajoute 973 mg d'iodure de sodium à 1,96 g du produit chloré obtenu au stade précédent en solution dans 15 $cm^3$ d'acétone puis agite 35 minutes à température ambiante. On évapore le solvant sous pression réduite, verse sur une solution aqueuse de thiosulfate de sodium à 10 %, sèche, évapore le solvant, purifie le résidu sur silice (éluant dichlorométhane-acétone 7-3, sous pression d'azote) et obtient 1,56 g de produit attendu.

STADE G : [6R(3(E), 6alpha, 7béta(Z))] iodure de 1-(3-(7-(((((éthoxyméthylphosphinyl) (3,4-bis((2-méthoxyéthoxy) méthoxy) phényl) méthoxy) imino) (2-((triphénylméthyl) amino) 4-thiazolyl) acétyl) amino) 2-(((4-méthoxyphényl) méthoxy) carbonyl) 8-oxo 5-thia 1-azabicyclo(4.2.0)oct-2-èn-3-yl) 2-propényl) quinoléinium

On dissout 700 mg du produit iodé obtenu au stade F et 700 mg de quinoléine dans 5 $cm^3$ de dichlorométhane puis évapore le solvant et agite à température ambiante pendant 1 heure.

On ajoute 20 $cm^3$ d'éther, agite 1 heure, essore le précipité et le rince à l'éther. Après purification sur silice (éluant dichorométhane-méthanol 95/5) on obtient 476 mg de produit attendu.
rf = 0,35 ($CH_2Cl_2$-méthanol 95-5).

Spectre RMN ($CDCl_3$ 300 MHz ppm)

| | |
|---|---|
| 1,1 à 1,29 | $-O-CH_2-\underline{CH_3}$ |
| 1,54 | $-P-CH_3$ |
| 3,01-3,06-3,38 | $OCH_3$ de -OMEM |
| 3,2 à 4,0 | $-O-\underline{CH_2}-CH_3$ et<br>$CH_2$ de OMEM et $CH_2S$ |
| 3,78 | $-\Phi-O-CH_3$ |
| 5,09 | -O-CH-P- |
| 6,0 à 6,14 | $-CH=CH-\underline{CH_2}$ |
| 6,4 à 6,5 | $-CH=\underline{CH}-CH_2$ |
| 6,7 | $H_5$ du thiazole |
| 6,85 à 7,40 | aromatiques |
| 7,96-8,14-8,27-8,38-9,0-10,33 | quinoléine. |

<u>STADE H</u> : Sel interne de (6R(3-(E), 6alpha, 7béta(Z))) 1-(3-(7-(((2-amino 4-thiazolyl) (éthoxyméthylphosphinyl (3,4-dihydroxyphényl) méthoxy) imino) amino) 2-carboxy 8-oxo 5-thia 1-azabicyclo(4.2.0)oct-2-èn-3-yl) 2-propényl) quinoléinium

On agite à température ambiante pendant 3 heures 450 mg de produit obtenu au stade G dans 4 $cm^3$ d'acide trifluoroacétique à 10 % d'anisole. On ajoute ensuite 0,4 $cm^3$ d'eau et agite encore 1 heure.

On filtre, rince à l'acide trifluoroacétique, ajoute à 0°C 25 $cm^3$ d'éther, agite 1 heure, essore, rince à l'éther et sèche sous pression réduite pendant 16 heures à température ambiante.

On obtient 255 mg de produit attendu.

Spectre RMN ($CDCl_3$ 300 MHz ppm)

| | |
|---|---|
| 1,0 à 1,2 | $-O-CH_2-\underline{CH_3}$ |
| 1,35 - 1,45 | $-P-CH_3$ |
| 3,08 à 3,9 | $-O-\underline{CH_2}-CH_3$ |
| 3,57-3,79 | $-S-CH_2$ |
| 5,2 à 5,28 | -O-CH-P- |
| 5,8 à 5,95 | $-CH=CH-\underline{CH_2}$ |
| 6,4 et 7,01 | $-CH=\underline{CH}-CH_2$ |
| 6,67 à 6,79 | $H_5$ du thiazole + aromatiques |
| 7,31 | $NH_2$ |
| 9,77 | -NH- |
| 8,07-8,26-8,51-8,52-9,34-9,59 | quinoléine. |

**EXEMPLE 2 : Sel interne de (6R(3-(E), 6alpha, 7béta(Z))) 7-(3-(7-(((2-amino 4-thiazolyl) (éthoxyméthylphosphinyl (3,4-dihydroxyphényl) méthoxy) imino) acétyl) amino) 2-carboxy 8-oxo 5-thia 1-azabicyclo(4.2.0)oct-2-èn-3-yl) 2-propényl) thiéno(2,3-b) pyridinium**

STADE A : [6R(3(E), 6alpha, 7béta(Z))] iodure de 1-(3-(7-(((((éthoxyméthylphosphinyl) (3,4-bis((2-méthoxyéthoxy) méthoxy) phényl) méthoxy) imino) (2-((triphénylméthyl) amino) 4-thiazolyl) acétyl) amino) 2-(((4-méthoxyphényl) méthoxy) carbonyl) 8-oxo 5-thia 1-azabicyclo(4.2.0)oct-2-èn-3-yl) 2-propényl) thiéno(2,3-b)pyridinium

On dissout 837 mg du dérivé iodé obtenu au stade F de l'exemple 1 et 608 mg de thiéno pyridine dans 5 $cm^3$ de dichlorométhane, puis évapore le solvant et agite à température ambiante pendant 1 heure.

On ajoute 20 $cm^3$ d'éther, agite 30 minutes, essore le précipité et le rince à l'éther. Après purification sur silice (éluant dichorométhane-méthanol 95/5) on obtient 693 mg de produit attendu.
rf = 0,55 ($CH_2Cl_2$-méthanol 80-20).
Spectre RMN ($CDCl_3$ 300 MHz ppm)

| | |
|---|---|
| 1,1 à 1,30 | -O-$CH_2$-$\underline{CH_3}$ |
| 1,40 à 1,65 | -P-$CH_3$ |
| 3,03 à 3,2-3,37 | $OCH_3$ de -OMEM |
| 3,28 à 4,2 | -O-$\underline{CH_2}$-$CH_3$ et -O-$CH_2$-O- de OMEM et $CH_2$S |
| 3,8 | -Φ-O-$CH_3$ |
| 5,1 à 5,35 | -O-$CH_2$-O- et $CO_2$-$CH_2$-Φ |
| 5,6 à 5,9 | -CH=CH-$\underline{CH_2}$ et -O-CH-P |
| 6,25 à 6,5 | -CH=$\underline{CH}$-$CH_2$ |
| 6,72 | $H_5$ du thiazole |
| 6,88 à 7,40 | aromatiques |
| 7,5 à 9,65 | thiénopyridine. |

STADE B : Sel interne de (6R(3-(E), 6alpha, 7béta(Z))) 7-(3-(7-(((2-amino 4-thiazolyl) (éthoxyméthylphosphinyl (3,4-dihydroxyphényl) méthoxy) imino) acétyl) amino) 2-carboxy 8-oxo 5-thia 1-azabicyclo(4.2.0)oct-2-èn-3-yl) 2-propényl) thiéno(2,3-b) pyridinium

On agite à température ambiante pendant 3 heures 693 mg de produit obtenu au stade A, dans 6 $cm^3$ d'acide trifluoroacétique à 10 % d'anisole. On ajoute ensuite 0,5 $cm^3$ d'eau et agite encore 1 heure.

On filtre, rince à l'acide trifluoroacétique, ajoute à 0°C 25 $cm^3$ d'éther, agite 45 minutes, essore, rince à l'éther et sèche sous pression réduite pendant 16 heures à température ambiante. On obtient 343 mg de produit attendu.
Spectre RMN ($CDCl_3$ 300 MHz ppm)

| | |
|---|---|
| 1,09 à 1,15 et 1,36-1,46 | -O-$CH_2$-$\underline{CH_3}$ et -P-$CH_3$ |
| 3,6 à 4,01 | -S-$CH_2$ et -O-$\underline{CH2}$-$CH_3$ |
| 5,25 | -O-CH-P |
| 5,68 | -CH=CH-$\underline{CH_2}$ |
| 6,33 | -CH=$\underline{CH}$-$CH_2$- |
| 6,68 à 6,80 | $H_5$ du thiazole + aromatiques |

| | |
|---|---|
| 7,83-8,27-8,16-9,09-9,24 | thiénopyridine. |

**EXEMPLE 3 : Sel interne de (6R(3-(E), 6alpha, 7béta(Z))) 7-(3-(7-(((2-amino 4-thiazolyl) ((hydroxyméthylphosphinyl (3,4-dihydroxyphenyl) méthoxy) imino) acétyl) amino) 2-carboxy 8-oxo 5-thia 1-azabicyclo(4.2.0)oct-2-èn-3-yl) 2-propényl) thiéno(2,3-b) pyridinium**

A 274 mg de produit obtenu à l'exemple 2, en suspension dans 10 $cm^3$ de dichlorométhane, on ajoute 495 mg de N-méthyl N-(triméthylsilyl) trifluoro acétamide (MSTFA) et agite 10 minutes à température ambiante.

On ajoute 427 mg d'iodure de triméthylsilyle puis agite 2 heures à température ambiante.

On ajoute 2 $cm^3$ de tétrahydrofuranne, agite 1 heure à température ambiante afin d'éliminer l'excès d'iodure de triméthylsilyle puis évapore le solvant, reprend le résidu dans 8 $cm^3$ d'acétone à 10 % de méthanol, agite 2 heures la suspension obtenue, essore, rince à l'éther, sèche sous pression réduite pendant 16 heures à température ambiante et obtient 215 ma de produit attendu.

Spectre RMN ($CDCl_3$ 300 MHz ppm)

| | |
|---|---|
| 1,32 | $-P-CH_3$ |
| 3,5 à 3,9 | $-S-CH_2$ |
| 5,06 | -O-CH-P |
| 6,31 et 7,15 | CH=CH-<u>$CH_2$</u> |
| 6,5 à 6,9 | $H_5$ du thiazole + aromatiques |
| 9,76 | NH |
| 7,88-8,28-8,14-9,08-9,22 | thiénopyridine. |

**EXEMPLE 4 : Sel interne de (6R(3-(E), 6alpha, 7béta(Z))) 7-(3-(7-(((2-amino 4-thiazolyl) (((2,5-dichloro 3,4-dihydroxyphényl) (diéthoxyphosphinyl) méthoxy) imino) acétyl) amino) 2-carboxy 8-oxo 5-thia 1-azabicyclo(4.2.0)oct-2-èn-3-yl) 2-propényl) thiéno(2,3-b) pyridinium**

STADE A : (hydroxy ((2,5-dichloro 3,4-bis((2-méthoxyéthoxy) méthoxy) phényl) méthyl) éthoxyphosphinate d'éthyle

On agite 3 heures à température ambiante 1,15 g de 2,5-dichloro 3,4-bis[(2-méthoxy éthoxy) méthoxy] benzaldéhyde préparé comme indiqué dans la demande de brevet européen EP 0551034, avec 0,42 g de diéthylphosphite et 2 g d'alumine. On reprend dans du dichlorométhane, filtre et évapore le solvant sous pression réduite. Après purification sur silice (éluant dichlorométhane acétate d'éthyle 5-5), on obtient 1,13 g de produit attendu.

Rf = 0,15 ($CH_2Cl_2$-AcOEt 7-3)

STADE B : ((2,5-dichloro 3,4-bis((2-méthoxyéthoxy) méthoxy) phényl) ((1,3-dihydro 1,3-dioxo-2H-isoindol 2-yl) oxy) méthyl) éthoxyphosphinate d'éthyle

On mélange sous atmosphère inerte, 421 mg de produit obtenu au stade A, 2 $cm^3$ de toluène, 393 mg de triphénylphosphine, 179 mg de N-hydroxy phtalimide puis refroidit cette suspension à 0°C et ajoute 0,23 $cm^3$ d'azodicarboxylate de diéthyle. On agite 1 heure à 0°C puis 2 heures à température ambiante, ajoute quelques $cm^3$ d'eau, extrait à l'acétate d'éthyle, lave à l'eau, sèche et évapore les solvants sous pression réduite. On obtient 0,85 g de produit brut que l'on purifie sur silice (éluant dichlorométhane-acétate d'éthyle 7-3) et obtient 310 mg de produit attendu.

Rf = 0,3 ($CH_2Cl_2$-AcOEt 7-3).

Spectre RMN ($CDCl_3$ 300 MHz ppm)

| | |
|---|---|
| 1,3-1,4 et 4,1 à 4,4 | $P-(OEt)_2$ |
| 3,3 et 3,37 | $CH_3$ de OMEM |
| 3,5 à 3,6-3,9 à 4,05 | $-O-CH_2-CH_2-O$ de OMEM |

| | |
|---|---|
| 5,18-5,25 | $-O-CH_2-O$ de OMEM |
| 6,25 | -CH~~ P |
| 8,01 | CH phényl |
| 7,65 à 7,80 | phtalimide. |

STADE C : (aminooxy ((2,5-dichloro 3,4-bis((2-méthoxyéthoxy) méthoxy) phényl) méthyl) éthoxyphosphinate d'éthyle

On ajoute 0,50 g d'hydrate d'hydrazine à 6 g de produit obtenu au stade B en solution dans 60 $cm^3$ d'éthanol puis agite 1 heure à température ambiante.

On filtre, évapore le solvant sous pression réduite, reprend le résidu dans quelques $cm^3$ de dichlorométhane, filtre, évapore le solvant et obtient 3,9 g de produit attendu.

rf = 0,15 ($CH_2Cl_2$-AcOEt 6-4)

Spectre RMN ($CDCl_3$ 300 MHz ppm)

| | |
|---|---|
| 1,28-1,32 et 4,0 à 4,25 | $P-(O-CH_2-CH_3)_2$ |
| 3,38 et 3,39-3,59 et 4,00 | OMEM |
| et 5,25 et 5,27 | |
| 3,8 à 4,3 | $-P-O-\underline{CH_2}-CH_3$ |
| 5,47 (d,J=15,5) | -CH~~ P |
| 7,45 | aromatiques. |

STADE D : (Z) acide α-((diéthoxyphosphinyl) (2,5-dichloro 3,4-((2-méthoxyéthoxy) méthoxy) phényl) méthoxy) imino) (2-((triphénylméthyl) amino) 4-thiazoleacétique.

On agite 1 heure à température ambiante 544 mg du produit obtenu au stade C en solution dans 5 $cm^3$ de méthanol et 570 mg d'acide oxo-[2-[(triphénylméthyl) amino] thiazol 4-yl] acétique (décrit dans la demande de brevet belge n° 864828.

On évapore le solvant sous pression réduite, purifie par chromatographie sur silice (éluant acétate d'éthyle/éthanol 7-3) et obtient 520 mg de produit attendu.

rf = 0,2 (AcOEt/EtOH 7-3)

Spectre RMN ($CDCl_3$ 300 MHz ppm)

| | |
|---|---|
| 1,16-1,26 et 4,10 | $-P-(O-CH_2-CH_3)_2$ |
| 3,35-3,36 | $CH_3$ de OMEM |
| 3,57-3,97 | $CH_2$ de OMEM |
| 5,22 (d,J=16,5) | -CH~~ P- |
| 6,70 | $H_5$ du thiazole |
| 7,92 | aromatiques. |

STADE E : [6R(3(E), 6alpha, 7béta(Z))] 3-(3-chloro 1-propényl) 7-(((((2,5-dichloro 3,4-bis((2-méthoxyéthoxy) méthoxy) phényl) (diéthoxyphosphinyl) méthoxy) imino) (2-((triphénylméthyl) amino) 4-thiazolyl) acétyl) amino) 8-oxo 5-thia 1-azabicyclo(4.2.0)oct-2-ène-2-carboxylate de (4-méthoxyphényl) méthyle

A une solution de 3,1 g d'acide obtenu au stade D dans 60 $cm^3$ de dichlorométhane, on ajoute 1,57 g de chlorhydrate de 7β-amino 3-[(Z) 3-chloro 1-propényl] 8-oxo 5-thia 1-azabicyclo[4.2.0]oct-2-ène 2-carboxylate de 4-méthoxy benzyle (décrit dans la demande de brevet européen n° 0333154) puis refroidit le milieu réactionnel à 0°C. On ajoute 763 mg de diméthylamino propyl-éthyl-carbodiimide (EDC) et poursuit l'agitation à 0°C pendant 1

heure. On reprend au dichlorométhane puis lave avec une solution de tampon phosphate pH 7, sèche et évapore le solvant sous pression réduite. On obtient après purification par chromatographie sur silice (éluant dichlorométhane-éther (85-15) 1,42 g de produit attendu.
rf = 0,2 ($CH_2Cl_2$-AcOEt 8-2).
Spectre RMN ($CDCl_3$ 300 MHz ppm).

| | |
|---|---|
| 1,09-1,31-3,98-4,18 | -P-(O-$CH_2$-$CH_3$)$_2$ |
| 3,44 | $CH_2$-S- |
| 3,37 | $CH_3$ de OMEM |
| 3,58 et 5,25 | $CH_2$ de O-MEM |
| 3,81 | méthoxybenzyle |
| 5,77-6,32 | -CH=CH-$CH_2$Cl |
| 6,21 | -CH $\sim$ P- |
| 6,81 à 6,94 | tritylamino |
| 7,30-7,62 | aromatiques. |

STADE F : [6R(3(E), 6alpha, 7béta(Z))] 3-(3-iodo 1-propényl) 7-(((((diéthoxyphosphinyl) 2,5-dichloro (3,4-bis((2-méthoxyéthoxy) méthoxy) phényl) méthoxy) imino) (2-((triphénylméthyl) amino) 4-thiazolyl) acétyl) amino) 8-oxo 5-thia 1-azabicyclo(4.2.0) oct-2-ène-2-carboxylate de (4-méthoxyphényl) méthyle

On ajoute 168 mg d'iodure de sodium et un cristal d'iode à 360 mg du produit chloré obtenu au stade précédent en solution dans 2 $cm^3$ d'acétone puis agite 1 heure à température ambiante. On évapore le solvant sous pression réduite, reprend dans du dichlorométhane, lave avec une solution aqueuse de thiosulfate de sodium à 10 %, sèche, évapore le solvant, et obtient 312 mg de produit attendu.

STADE G : [6R(3(E), 6alpha, 7béta(Z))] iodure de 7-(3-(7-(((((2,5-dichloro 3,4-bis((2-méthoxyéthoxy) méthoxy) phényl) (diéthoxyphosphinyl) méthoxy) imino) (2-((triphénylméthyl) amino) 4-thiazolyl) acétyl) amino) 2-(((4-méthoxyphényl) méthoxy) carbonyl) 8-oxo 5-thia 1-azabicyclo(4.2.0)oct-2-èn-3-yl) 2-propényl) thiéno(2,3-b)pyridinium.

On agite 1 heure à température ambiante 260 mg de dérivé iodé obtenu au stade F et 240 mg de thiénopyridine. On précipite par addition d'éther, essore et sèche sous pression réduite à température ambiante. On chromatographie sur silice (éluant dichlorométhane-méthanol 95-5) et obtient 110 mg de produit attendu.
rf = 0,25 ($CH_2Cl_2$-MeOH 9-1)
Spectre RMN ($CDCl_3$ 400 MHz ppm)

| | |
|---|---|
| 1,07-1,12-1,25 à 1,28-3,69 | -P-(O-$CH_2$-$CH_3$)$_2$ |
| 3,36-3,37 | $OCH_3$ de -OMEM |
| 3,23 à 3,28 et 3,57-3,97 | $CH_2$ de OMEM |
| 3,80 et 6,9 à 7,38 | -Φ-O-$CH_3$ |
| 4,16 | -$CH_2$-S- |
| 6,20 | -O-CH-P- |
| 6,44 | -CH=<u>CH</u>-$CH_2$- |
| 6,76-6,80 | $H_5$ du thiazole |
| 7,28 et 7,54-7,60 | NH-trityle |

| | |
|---|---|
| 7,68-7,87-8,06-8,82-8,98 | thiénopyridine |
| 8,57 | CO-NH. |

STADE H : Sel interne de (6R(3-(E), 6alpha, 7béta(Z))) 7-(3-(7-(((2-amino 4-thiazolyl) (((2,5-dichloro 3,4-dihydroxyphényl) (diéthoxyphosphinyl) méthoxy) imino) acétyl) amino) 2-carboxy 8-oxo 5-thia 1-azabicyclo(4.2.0) oct-2-èn-3-yl) 2-propényl) thiéno(2,3-b) pyridinium

On agite à température ambiante pendant 2 heures 100 mg de produit obtenu au stade G dans 1 $cm^3$ d'acide trifluoroacétique à 10 % d'anisole.

On filtre, rince à l'acide trifluoroacétique et précipite le filtrat par addition d'éther. On essore, lave à l'éther et sèche sous pression réduite. On obtient 43 mg de produit attendu.

Spectre RMN ($CDCl_3$ 400 MHz ppm)

| | |
|---|---|
| 1,28 et 3,93 à 4,10 | P-(O-$CH_2$-$CH_3$)$_2$ |
| 3,60 à 3,80 | -S-$CH_2$ |
| 5,76 | -CH-P- |
| 5,67 | -CH=CH-<u>$CH_2$</u>-$N^+$ |
| 6,31 | -CH=<u>CH</u>-$CH_2$-$N^+$ |
| 6,76 | $H_5$ thiazole |
| 7,02 | aryle dichloré |
| 7,15 | -<u>CH</u>=CH-$CH_2$-$N^+$ |
| 7,88-8,14-8,27-9,08-9,22 | thiéno pyridine. |

**EXEMPLE 5 : Sel interne de (6R(3-(E), 6alpha, 7béta(Z))) 7-(3-(7-(((2-amino 4-thiazolyl) (((2,5-dichloro 3,4-dihydroxyphényl) phosphonométhoxy) imino) acétyl) amino) 2-carboxy 8-oxo 5-thia 1-azabicyclo(4.2.0) oct-2-èn-3-yl) 2-propényl) thiéno(2,3-b) pyridinium**

A 190 mg de produit obtenu à l'exemple 4, en suspension dans 4 $cm^3$ de dichlorométhane, on ajoute 0,21 $cm^3$ de N-méthyl N-(triméthylsilyl) trifluoro acétamide (MSTFA) et agite 5 minutes à température ambiante.

On ajoute 0,3 $cm^3$ d'iodure de triméthylsilyle puis agite 2 heures à température ambiante.

On ajoute 2 $cm^3$ de tétrahydrofuranne, agite 15 minutes à température ambiante afin d'éliminer l'excès d'iodure de triméthylsilyle puis évapore le solvant, reprend le résidu dans 3 $cm^3$ d'acétone à 10 % de méthanol, agite 45 minutes à température ambiante, évapore le solvant sous pression réduite et concrétise le produit par addition d'éther. On filtre, lave à l'éther et sèche sous pression réduite, on obtient 123 mg de produit attendu.

Spectre RMN (DMSO 400 MHz ppm)

| | |
|---|---|
| 5,70 à 5,85 | -O-CH-P |
| 5,67 | CH=CH-<u>$CH_2$</u> |
| 6,33 | CH-<u>CH</u>-$CH_2$ |
| 6,77 | $H_5$ du thiazole |
| 7,11 à 7,15 | <u>CH</u>=CH-$CH_2$ et chlorophényl |
| 7,89 et 8,28-8,15-9,09-9,22 | thiénopyridine |
| 9,61 et 9,71 | NH. |

**EXEMPLE 6 : Sel interne de (6R(3-(E), 6alpha, 7béta(Z)) 7-(3-(7-(((2-amino 4-thiazolyl) (((diéthoxyphosphinyl) 3,4-dihydroxyphényl) méthoxy) imino) acétyl) amino) 2-carboxy 8-oxo 5-thia 1-azabicyclo(4.2.0)oct-2-èn-3-yl) 2-propényl) thiéno(2,3-b) pyridinium**

STADE A : (hydroxy (3,4-bis((2-méthoxyéthoxy) méthoxy) phényl) méthyl) éthoxyphosphinate d'éthyle

On ajoute à température ambiante 30 g d'alumine à 15,7 g de 3,4-bis[(2-méthoxyéthoxy) méthoxy] benzaldéhyde en solution dans 6,9 g de diéthylphosphite. On laisse 2 heures à température ambiante, filtre, lave au dichlorométhane, évapore le solvant sous pression réduite et récupère 21 g de produit brut que l'on chromatographie sur silice (éluant dichlorométhane puis acétate d'éthyle-éthanol 9-1). On obtient 19,4 g de produit attendu.
rf = 0,25 (AcOEt-EtOH 9-1).

STADE B ((3,4-bis((2-méthoxyéthoxy) méthoxy) phényl) ((1,3-dihydro 1,3-dioxo-2H-isoindol-2-yl) oxy) méthyl) éthoxyphosphinate d'éthyle

On mélange sous atmosphère inerte, 12 g de produit obtenu au stade A, 240 $cm^3$ de tétrahydrofuranne, 10,35 g de triphénylphosphine, 4,76 g de N-hydroxy phtalimide puis refroidit cette solution à 0°C et ajoute 6,2 $cm^3$ d'azadicarboxylate de diéthyle dans 10 $cm^3$ de tétrahydrofuranne. On agite 30 minutes à 0°C puis 30 minutes à température ambiante, ajoute 100 $cm^3$ d'eau, extrait à l'acétate d'éthyle, lave à l'eau, sèche et évapore les solvants sous pression réduite, abandonne 16 heures à +4°C, essore les cristaux formés et les lave à l'éther, évapore le solvant du filtrat et chromatographie le résidu sur silice (éluant acétate d'éthyle puis acétate d'éthyle-ethanol 95-5). On obtient 6,8 g de produit attendu.
rf = 0,3 (AcOEt-EtOH 9-1)
Spectre RMN ($CDCl_3$ 250 MHz ppm)

| | |
|---|---|
| 1,33-1,37 et 4,25 | $-P-(OEt)_2$ |
| 3,34-3,36 | les $CH_3$ de OMEM |
| 3,55-3,88 | les $CH_2$ de OMEM |
| 5,48 | -CH ~ P- |
| 7,10 à 7,8 | aromatiques. |

STADE C : (aminooxy (3,4-bis((2-méthoxyéthoxy) méthoxy) phényl) méthyl) éthoxyphosphinate d'éthyle

On agite 1 heure à 60°C 6,8 g de produit préparé au stade B, 100 $cm^3$ d'éthanol et 0,95 $cm^3$ d'hydrate d'hydrazine. On refroidit, filtre, lave à l'éthanol et évapore le solvant sous pression réduite. On chromatographie le résidu sur silice (éluant acétate d'éthyle-éthanol 95-5) et obtient 3,9 g de produit attendu.
rf = 0,15 (AcOEt-EtOH 95-5)
Spectre RMN ($CDCl_3$ 250 MHz ppm)

| | |
|---|---|
| 1,25-1,30 et 4,10 | $-P-(OEt)_2$ |
| 3,36-3,38 | $CH_3$ de OMEM |
| 3,56-3,85 | $(CH_2)_2$ de OMEM |
| 4,89 | -CH ~ P- |
| 5,32 | $O-CH_2-O$ de OMEM |
| 7,06 et 7,2 à 7,3 | aromatiques. |

STADE D : [6R(3(E), 6alpha, 7béta(Z))] iodure de 1-(3-(7-(((((diéthoxyphosphinyl) (3,4-bis((2-méthoxyéthoxy) méthoxy) phényl) méthoxy) imino) (2-((triphénylméthyl) amino) 4-thiazolyl) acétyl) amino) 2-(((4-méthoxyphényl) méthoxy) carbonyl) 8-oxo 5-thia 1-azabicyclo(4.2.0)oct-2-èn-3-yl) 2-propényl) quinoléinium

On dissout 373 mg du produit obtenu au stade C et 650 mg de [6R(3(E), 6alpha, 7béta(Z))] iodure de 1-(3-(7-((((hydroxy imino) (2-((triphénylméthyl) amino) 4-thiazolyl) acétyl) amino) 2-(((4-méthoxyphényl) méthoxy) carbonyl) 8-oxo 5-thia 1-azabicyclo(4.2.0)oct-2-èn-3-yl) 2-propényl) quinoléinium dans 6 $cm^3$ de dichlorométhane et 20 $cm^3$ de méthanol et ajoute 150 mg d'acide paratoluène sulfonique. On agite 16 heures à température ambiante, ajoute de nouveau 100 mg du produit obtenu au stade C et maintient 6 heures sous agitation à température ambiante. On évapore le solvant sous pression réduite, reprend le résidu dans l'éther, sèche sous pression réduite et récupère 905 mg de produit brut que l'on utilise tel quel pour le stade suivant.

STADE E : Sel interne de (6R(3-(E), 6alpha, 7béta(Z)) 7-(3-(7-(((2-amino 4-thiazolyl) (((diéthoxyphosphinyl) 3,4-dihydroxyphényl) méthoxy) imino) acétyl) amino) 2-carboxy 8-oxo 5-thia 1-azabicyclo(4.2.0)oct-2-èn-3-yl) 2-propényl) thiéno(2,3-b) pyridinium

On mélange à température ambiante, 900 mg de produit obtenu au stade D dans 9 $cm^3$ d'acide trilfuoroacétique à 10 % d'anisole. On ajoute ensuite 0,9 $cm^3$ de dichlorométhane et agite 2 heures.

On évapore le solvant sous pression réduite, ajoute de l'éther, essore le précipité, le lave à l'éther et le sèche. On le reprend de nouveau dans 7 $cm^3$ d'acide trifluoroacétique à 15 % d'eau et 0,6 $cm^3$ de dichlorométhane, agite 2 heures, filtre, évapore le solvant sous pression réduite, précipite à l'éther, essore, sèche et recueille 519 mg de produit attendu.
rf = 0,3 (acétone-eau 7-3).
Spectre RMN (DMSO 300 MHz ppm)

| | |
|---|---|
| 1,05 à 1,20 et 3,45 à 4,0 | $-P-(OEt)_2$. |

**EXEMPLE 7 : Sel interne de (6R(3-(E), 6alpha, 7béta(Z))) 7-(3-(7-(((2-amino 4-thiazolyl) (3,4-dihydroxyphényl) phosphonométhoxy) imino) acétyl) amino) 2-carboxy 8-oxo 5-thia 1-azabicyclo (4.2.0)oct-2-èn-3-yl) 2-propényl) thiéno(2,3-b) pyridinium**

A 500 mg de produit obtenu à l'exemple 6, en suspension dans 10 $cm^3$ de dichlorométhane, on ajoute 0,5 $cm^3$ de N-méthyl N-(triméthylsilyl) trifluoro acétamide (MSTFA) et agite 5 minutes à température ambiante.

On ajoute 1 $cm^3$ d'iodure de triméthylsilyle puis agite 2 heures à température ambiante.

On ajoute 2,5 $cm^3$ de tétrahydrofuranne, agite 1 heure à température ambiante afin d'éliminer l'excès d'iodure de triméthylsilyle puis évapore le solvant, reprend le résidu dans 10 $cm^3$ d'acétone à 10 % de méthanol, agite 2 heures la suspension obtenue, essore, rince au dichlorométhane puis à l'éthanol, sèche sous pression réduite à température ambiante et obtient 296 mg de produit attendu.
Spectre RMN ($CDCl_3$ 300 MHz ppm)

| | |
|---|---|
| 1,32 | $-P-CH_3$ |
| 3,61 à 3,77 | $-S-CH_2$ |
| 5,11 | -O-CH-P |
| 5,67-6,31 et 7,18 | $CH=CH-CH_2-$ |
| 6,6 à 6,9 | $H_5$ du thiazole + aromatiques |
| 7,89-8,26-8,15-9,08-9,23 | thiénopyridine. |

**EXEMPLE 8 : Sel interne de (6R(3-(E), 6alpha, 7béta(Z))) 7-(3-(7-(((2-amino 4-thiazolyl) (((éthoxyméthylphosphinyl) (2,5-dichloro (3,4-dihydroxyphényl) méthoxy) imino) acétyl) amino) 2-carboxy 8-oxo 5-thia 1-azabicyclo(4.2.0)oct-2-èn-3-yl) 2-propényl) thiéno(2,3-b) pyridinium (isomère A)**

Stade A : (hydroxy 2,5-dichloro 3,4-bis ((2-méthoxyéthoxy) méthoxy) phényl) méthyl) méthyl phosphinate d'éthyle.

On chauffe à 70°C pendant 70 minutes 29,97 g de 2,5-dichloro 3,4-bis ((méthoxyéthoxy) méthoxy benzaldéhyde préparé comme indiqué dans la demande de brevet européen EP 0 551 034, 6,8 g de réactif méthyléthylphosphite préparé comme indiqué dans le stade A de l'exemple 1, en présence de 16,68 ml de triéthylamine. On élimine la triéthylamine sous pression réduite et obtient 30,5 g de produit attendu que l'on purifie par chromatographie sur silice (éluant : AcOEt-EtOH 98-2). rf ≈ 0,35 (AcOEt-EtOH 98-2).

Stade B : ((2,5-dichloro 3,4-bis ((2-méthoxyéthoxy) méthoxy) phényl) ((1,3-dihydro 1,3-dioxo 2H-isoindol 2-yl) oxy) méthyl) méthylphosphinate d'éthyle (isomère A et isomère B).

On opère comme au stade B de l'exemple 1 en utilisant au départ 18,4 g de produit obtenu au stade A, 6,72 g d'hydroxyphtalimide, 14,72 g de triphénylphosphine et 8,89 ml de diéthylazodicarboxylate et en effectuant l'extraction à l'acétate d'éthyle. On obtient 50 g de produit brut que l'on purifie par chromatographie sur silice (éluant : AcOEt-hexane 9-1) et obtient 7,23 g d'isomère A rf = 0,45 (AcOEt-hexane 9-1) et 8,78 g d'isomère B rf = 0,40 (AcOEt-hexane 9-1).

Spectre RMN ($CDCl_3$ 300 MHz ppm)

| | |
|---|---|
| 1,28 et 3,90 à 4,22 | P-(OEt) |
| 1,81 | P-($CH_3$) |
| 3,33 et 3,37 | $CH_3$ de OMEM |
| 3,55-3,98 | O-$CH_2$-$CH_2$-O de OMEM |
| 5,19-5,25 | -O-$CH_2$-O de OMEM |
| 6,27 | -CH ~ P |
| 8,00 | CH phényl |
| 7,77 | phtalimide. |

Stade C : (amino oxy ((2,5-dichloro 3,4-bis ((2-méthoxyéthoxy) méthoxy) phényl) méthyl) méthylphosphinate d'éthyle (isomère A).

On refroidit à 0°C 7,2 g de produit obtenu au stade B (isomère A) dans 60 ml de dichlorométhane et ajoute 0,824 ml d'hydrate d'hydrazine et agite 90 minutes. On filtre, évapore le solvant sous pression réduite,chromatographie le résidu sur silice (éluant : AcOEt-EtOH 95-5) et récupère 5,29 g de produit attendu. rf = 0,20 (AcOEt-EtOH 95-5).

Spectre RMN ($CDCl_3$ 300 MHz ppm)

| | |
|---|---|
| 1,24 et 3,83 à 4,16 | P-(OEt) |
| 1,54 (d, J=14,5) | P-($CH_3$) |
| 3,38 et 3,39-3,58 et 4,01 | OMEM |
| et 5,26 et 5,27 | |
| 5,41 (d, J=9,5) | -CH ~ P |
| 5,78 | $NH_2$ |
| 7,41 | aromatiques. |

Stade D : (Z) acide $\alpha$-((méthyléthoxyphosphinyl) (2,5-dichloro 3,4-((2-méthoxyéthoxy) méthoxy) phényl) méthoxy) imino) (2-((triphénylméthyl) amino) 4-thiazole acétique (isomère A).

On opère comme au stade D de l'exemple 1 en utilisant au départ 4 g de l'oxyamine obtenue au stade C et 3,60 g d'acide oxo (2-((triphénylméthyl) amino) thiazol 4-yl) acétique (décrit dans la demande de brevet belge n° 864828. On obtient 7,40 g de produit brut que l'on chromatographie sur silice (éluant : $CH_2Cl_2$-MeOH 95-5 puis 90-10). On récupère 5,69 g de produit attendu. rf = 0,5 ($CH_2Cl_2$-MeOH 85-15).

Spectre RMN ($CDCl_3$ 300 MHz ppm)

| | |
|---|---|
| 1,03 | $CH_3$ de P-(OEt) |
| 1,63 | P-($CH_3$) |

| | |
|---|---|
| 3,45-3,50 | $CH_3$ de OMEM |
| 3,54-3,64-3,79-3,95 | $CH_2$ de P-(OEt) et de O-$(CH_2)_2$-O |
| 5,20 | O-$CH_2$-O |
| 6,55 | $H_5$ thiazole |
| 7,20-7,53 | aromatiques. |

Stade E : (6R(3(E), 6alpha, 7béta(Z))) 3-(3-chloro 1-propényl) 7-(((((2,5-dichloro 3,4-bis ((2-méthoxyéthoxy) méthoxy) phényl) (méthyléthoxyphosphinyl) méthoxy) imino) 2-((triphénylméthyl) amino) 4-thiazolyl) acétyl) amino) 8-oxo 5-thia 1-azabicyclo(4.2.0)oct-2-èn-2-carboxylate de (4-méthoxyphényl) méthyle (isomère A).

On opère comme à l'exemple 1 stade E en utilisant au départ 5,60 g de produit obtenu au stade D et 2,68 g de chlorhydrate de 7béta-amino 3-((Z) 3-chloro 1-propényl) 8-oxo 5-thia 1-azabicyclo(4.2.0)oct-2-èn-2-carboxylate de 4-méthoxy benzyle (décrit dans la demande de brevet européen n° 0333 154) et 1,43 g de diméthylaminopropyléthyl carbodiimide (EDC). On obtient 7,56 g de produit brut que l'on purifie par chromatographie sur silice (éluant : $CH_2Cl_2$-Acétone 85-15). rf = 0,3 ($CH_2Cl_2$-Acétone 85-15).
Spectre RMN ($CDCl_3$ 300 MHz ppm)

| | |
|---|---|
| 1,00-1,10 | P-(O-$CH_2$-$CH_3$) |
| 3,44 | $CH_2$-S- |
| 3,37 | $CH_3$ de OMEM |
| 3,40 à 4 | $(CH_2)_2$ de O-MEM et $CH_2$-Cl |
| 3,81 | méthoxybenzyle |
| 5,12à 5,38 | O-$CH_2$-O |
| 6,31 | -CH=<u>CH</u>-$CH_2$Cl |
| 6,15 à 6,18 | -CH ~ P- |
| 7,30 | tritylamino |
| 7,30-7,61 | aromatiques. |

Stade F : (6R(3(E), 6alpha, 7béta(Z))) 3-(3-iodo 1-propényl) 7-(((((2,5-dichloro 3,4-bis ((2-méthoxyéthoxy) méthoxy) phényl) (méthyléthoxyphosphinyl) méthoxy) imino) 2-((triphénylméthyl) amino) 4-thiazolyl) acétyl) amino) 8-oxo 5-thia 1-azabicyclo(4.2.0)oct-2-èn-2-carboxylate de (4-méthoxyphényl) méthyle (isomère A).

On opère comme au stade F de l'exemple 1 en utilisant au départ 1,47 g de produit préparé au stade E et 690 mg d'iodure de sodium. Après chromatographie sur silice (éluant: $CH_2Cl_2$-Acétone 80-20), on récupère 1,42 g de produit attendu utilisé tel quel pour le stade suivant.
Spectre RMN ($CDCl_3$ 300 MHz ppm)

| | |
|---|---|
| 1,00-1,09 et 3,99 | P-(OEt) |
| 1,65 à 1,71 et 3,47 | P-$CH_3$ et S-$CH_2$- |
| 3,37-3,38 | O-$CH_3$ de OMEM |
| 3,57-4,00 | 0-$CH_2$ de OMEM |
| 3,82 | Φ-$OCH_3$ |

| | |
|---|---|
| 5,10-5,25 | O-$CH_2$-O de OMEM et Φ-$CH_2$-O |
| 6,05 à 6,30 | O-CH-P et I-$CH_2$-CH |
| 6,79-6,82 | $H_5$ thiazole |
| 7,27 à 7,40 | NH-trityle. |

Stade G : (6R(3(E), 6alpha, 7béta(Z))) iodure de 7-(3-(7-(((((2,5-dichloro 3,4-bis ((2-méthoxyéthoxy) méthoxy) phényl) (méthyléthoxyphosphinyl) méthoxy) imino) 2-((triphénylméthyl) amino) 4-thiazolyl) acétyl) amino) 2-(((4-méthoxyphényl) méthoxy) carbonyl) 8-oxo 5-thia 1-azabicyclo(4.2.0)oct-2-èn-3-yl) 2-propényl) thiéno (2,3-b)pyridinium (isomère A).

On opère comme au stade G de l'exemple 4 en utilisant le produit obtenu au stade F et 0,776 de 2,3-b thiénopyridine. Après chromatographie sur silice (éluant : dichlorométhaneméthanol 90-10), on obtient 0,93 g de produit attendu.
rf = 0,25 ($CH_2Cl_2$-MeOH 95-5).
Spectre RMN ($CDCl_3$ 300 MHz ppm)

| | |
|---|---|
| 0,99-1,09 et | P-(O-$CH_2$-$CH_3$) |
| 3,57-3,98-3,40 à 4,00 | |
| 1,65-1,70 | P-$CH_3$ |
| 3,31-3,37 | $CH_3$ de OMEM et $CH_2$-S |
| 3,57-3,38 et 3,40 à 4,00 | $CH_2$ de O-MEM |
| 3,31-3,37 | -Φ-O-$CH_3$ |
| 4,16 | -$CH_2$-S- |
| 5,63-5,79-5,99 | $CH_2$ du propényl |
| 6,13-6,16 | -O-CH-P- |
| 6,49 | -CH=<u>CH</u>-$CH_2$- |
| 6,78-6,80 | $H_5$ du thiazole |
| 6,91 | les NH |
| 7,30 | NH-trityle |
| 7,51-7,60-7,65-7,88-8,06-8,82 | thiénopyridine. |

Stade H : sel interne de (6R(3(E), 6alpha, 7béta(Z))) 7-(3-(7-(((2-amino 4-thiazolyl) (((2,5-dichloro 3,4-dihydroxyphényl) (méthyléthoxyphosphinyl) méthoxy) imino) acétyl) amino) 2-carboxy 8-oxo 5-thia 1-azabicyclo(4.2.0) oct-2-èn-3-yl) 2-propényl) thiéno (2,3-b)pyridinium (isomère A).

On opère comme au stade H de l'exemple 1 en utilisant au départ 880 mg de produit obtenu au stade G et 9 ml de solution d'acide trifluoroacétique à 10% d'anisole. On obtient 476 mg de produit attendu.
Spectre RMN ($CDCl_3$ 400 MHz ppm)

| | |
|---|---|
| 1,16 à 3,97 | P-(O-$CH_2$-$CH_3$) |
| 1,48 | P-$CH_3$ |
| 3,55 à 3,80 | $CH_2$-S |

| | |
|---|---|
| 5,68 | -CH-P |
| 5,68 | -CH=CH-<u>CH</u>$_2$-N$^+$ |
| 6,35 | -CH=<u>CH</u>-CH$_2$-N$^+$ |
| 6,75-6,77 | $H_5$ du thiazole |
| 6,99 | aryle dichloré |
| 7,17 | -<u>CH</u>=CH-CH$_2$-N$^+$ |
| 7,30 | $NH_2$ |
| 7,89-8,29-9,09-9,15-9,23 | thiénopyridine |

**<u>EXEMPLE 9</u> : Sel interne de (6R(3-(E), 6alpha, 7béta(Z))) 7-(3-(7-(((2-amino 4-thiazolyl) (((éthoxyméthylphosphinyl) (2,5-dihydro (3,4-dihydroxyphényl) méthoxy) imino) acétyl) amino) 2-carboxy 8-oxo 5-thia 1-azabicyclo(4.2.0)oct-2-èn-3-yl) 2-propényl) thiéno(2,3-b) pyridinium (isomère B)**

<u>Stade A</u> : (amino oxy ((2,5-dichloro 3,4-bis ((2-méthoxyéthoxy) méthoxy) phényl) méthyl) méthylphosphinate d'éthyle (isomère B).

On opère comme au stade C de l'exemple 8 en utilisant au départ 9,5 g de l'isomère B obtenu comme au stade B de l'exemple 8 et 1,45 ml d'hydrate d'hydrazine. On obtient 6,70 g de produit attendu. F < 50°C.
Spectre RMN ($CDCl_3$ 300 MHz ppm)

| | |
|---|---|
| 1,30-4,12 | P-(OEt) |
| 1,45 | P-$CH_3$ |
| 3,60-4,01 | O-$CH_2$-$CH_2$-O |
| 5,27 | O-$CH_2$-O |
| 5,40 | CH〜P |

<u>Stade B</u> : (Z) acide α-((éthoxyméthylphosphinyl) 2,5-dichloro (3,4-((2-méthoxyéthoxy) méthoxy) phényl) méthoxy) 2-((triphénylméthyl) amino) 4-thiazole acétique (isomère B).

On opère comme au stade D de l'exemple 1 en utilisant au départ 4 g de produit obtenu au stade A ci-dessus et 3,60 g du dérivé cétoacide. On obtient 7,14 g de produit attendu.
rf = 0,50 ($CH_2Cl_2$-MeOH 85-15)
Spectre RMN ($CDCl_3$ 300 MHz ppm)

| | |
|---|---|
| 3,38 | $OCH_3$ de OMEM |
| 3,58-3,99-3,90 à 4,25 | O-$CH_2$-$CH_2$-O- |
| 5,23 | O-$CH_2$-O |
| 7,21 | trityle |
| 2,95 | 2H mobiles |
| 6,87 | 1H mobile |

<u>Stade C</u> : (6R(3(E), 6alpha, 7béta(Z))) 3-(3-chloro 1-propényl) 7-(((((éthoxyméthylphosphinyl) 2,5-dichloro 3,4-bis

((2-méthoxyéthoxy) méthoxy) phényl) méthoxy) imino) 2-((triphénylméthyl) amino) 4-thiazolyl) acétyl) amino) 8-oxo 5-thia 1-azabicyclo(4.2.0)oct-2-èn-2-carboxylate de (4-méthoxyphényl) méthyle (isomère B).

On opère comme à l'exemple 1 stade E en utilisant au départ 5,50 g de produit obtenu au stade B ci-dessus et 2,63 g de chlorhydrate de 7béta-amino 3-((Z) 3-chloro 1-propényl) 8-oxo 5-thia 1-azabicyclo(4.2.0)oct-2-èn-2-carboxylate de 4-méthoxy benzyle (décrit dans la demande de brevet européen n 0333 154) et 1,40 g de diméthylaminopropyléthyl carbodiimide (EDC). On obtient 7,32 g de produit brut que l'on purifie par chromatographie sur silice (éluant : $CH_2Cl_2$-Acétone 85-15). rf = 0,3 ($CH_2Cl_2$-Acétone 85-15).

Spectre RMN ($CDCl_3$ 300 MHz ppm)

| | |
|---|---|
| 1,27-1,42 | $CH_3$ de P-(OEt) et P-$CH_3$ |
| 3,38 | $CH_3$ de OMEM |
| 3,70 à 3,99 | $CH_2$-S |
| 3,81 | méthoxybenzyle |
| 3,98 à 3,99 | $(CH_2)_2$ de OMEM |
| 4,17 | -CH=CH-$\underline{CH_2}$Cl |
| 5,15-6,32 | -$\underline{CH}$=$\underline{CH}$-$CH_2$Cl |
| 6,16-6,18 | -CH $\sim$ P- |
| 6,78-6,84 | $H_5$ thiazole |
| 7,30 | tritylamino |
| 6,88-7,30 | aromatiques |
| 7,58 | dichlorophényle. |

<u>Stade D</u> : (6R(3(E), 6alpha, 7béta(Z))) 3-(3-iodo 1-propényl) 7-(((((éthoxyméthylphosphinyl) 2,5-dichloro 3,4-bis ((2-méthoxyéthoxy) méthoxy) phényl) méthoxy) imino) 2-((triphénylméthyl) amino) 4-thiazolyl) acétyl) amino) 8-oxo 5-thia 1-azabicyclo(4.2.0)oct-2-èn-2-carboxylate de (4-méthoxyphényl) méthyle (isomère B).

On opère comme au stade F de l'exemple 1 en utilisant au départ 2,98 g de produit obtenu au stade C ci-dessus et 1,40 g d'iodure de sodium. On obtient 3,14 g de produit attendu utilisé tel quel pour le stade suivant.

Spectre RMN ($CDCl_3$ 300 MHz ppm)

| | |
|---|---|
| 0,97-1,06 et 3,99 | P-(OEt) |
| 1,71 | P-$CH_3$ |
| 3,57 | O-$CH_3$ de OMEM |
| 3,58 | $CH_2$ de OMEM |
| 3,82 | $\Phi$-$OCH_3$ |
| 5,25 | O-$CH_2$-O de OMEM et $\Phi$-$CH_2$-O |
| 6,10 à 6,18 | O-CH-P |
| 6,77-6,80 | $H_5$ thiazole |
| 7,20 à 7,40 | NH-trityle. |

Stade E : (6R(3(E), 6alpha, 7béta(Z))) iodure de 7-(3-(7-(((((2,5-dichloro 3,4-bis ((2-méthoxyéthoxy) méthoxy) phényl) (méthyléthoxyphosphinyl) méthoxy) imino) 2-((triphénylméthyl) amino) 4-thiazolyl) acétyl) amino) 2-(((4-méthoxyphényl) méthoxy) carbonyl) 8-oxo 5-thia 1-azabicyclo(4.2.0)oct-2-èn-3-yl) 2-propényl) thiéno (2,3-b)pyridinium (isomère B).

On opère comme au stade G de l'exemple 4 en utilisant le produit obtenu au stade D et 1,57 g de 2,3-b thiénopyridine. Après chromatographie sur silice (éluant : dichlorométhaneméthanol 90-10), on obtient 1,92 g de produit attendu.
rf = 0,25 ($CH_2Cl_2$-MeOH 95-5).
Spectre RMN ($CDCl_3$ 300 MHz ppm)

| | |
|---|---|
| 1,20 à 1,40 et 4,14 | P-(OEt) |
| 1,20 à 1,40 | P-$CH_3$ |
| 3,31-3,37 | $CH_3$ de OMEM et $CH_2$-S |
| 3,57-3,99-3,66 | O-$CH_2$-$CH_2$-O et S-$CH_2$ |
| 3,37-3,38 | -Φ-O-$CH_3$ |
| 5,25 | O-$CH_2$-O |
| 5,60 à 6,04 et 6,44 | $N^+$-$CH_2$-CH |
| 6,75-6,79 | $H_5$ du thiazole |
| 6,90 à 7,40 | N-trityle et Φ-$CH_2$-O |
| 7,50 à 7,53-7,70-7,87-8,06- | thiénopyridine. |
| 8,83-9,97 | |

Stade F : sel interne de (6R(3(E), 6alpha, 7béta(Z))) 7-(3-(7-(((2-amino 4-thiazolyl) (((2,5-dichloro 3,4-dihydroxyphényl) (méthyléthoxyphosphinyl) méthoxy) imino) acétyl) amino) 2-carboxy 8-oxo 5-thia 1-azabicyclo(4.2.0) oct-2-èn-3-yl) 2-propényl) thiéno (2,3-b)pyridinium (isomère B).

On opère comme au stade H de l'exemple 1 en utilisant au départ 1,9 g de produit obtenu au stade G et 19 ml de solution d'acide trifluoroacétique à 10% d'anisole. On obtient 818 mg de produit attendu.
Spectre RMN ($CDCl_3$ 400 MHz ppm)

| | |
|---|---|
| 1,15 et 3,55 à 4,10 | P-(O-$CH_2$-$CH_3$) |
| 1,53 | P-$CH_3$ |
| 3,55 à 4,10 | $CH_2$-S |
| 5,68 | -CH-P |
| 5,68 | -CH=CH-<u>$CH_2$</u>-$N^+$ |
| 6,34 | -CH=<u>CH</u>-$CH_2$-$N^+$ |
| 6,76 | $H_5$ du thiazole |
| 6,99 | aryle dichloré |
| 7,17 | -<u>CH</u>=CH-$CH_2$-$N^+$ |
| 7,29 | $NH_2$ |

| | |
|---|---|
| 7,89-8,10-8,28-9,08 | thiénopyridine |

**<u>EXEMPLE 10</u> : Sel interne de (6R(3-(E), 6alpha, 7béta(Z))) 7-(3-(7-(((2-amino 4-thiazolyl) (((hydroxyméthylphosphinyl) (2,5-dichloro (3,4-dihydroxyphényl) méthoxy) imino) acétyl) amino) 2-carboxy 8-oxo 5-thia 1-azabicyclo(4.2.0)oct-2-èn-3-yl) 2-propényl) thiéno(2,3-b) pyridinium**

On mélange sous atmosphère inerte 80 mg de produit obtenu à l'exemple 8 ou 9 ou leur mélange et 88 µl de N-méthyl N-triméthylsilyl trifluoroacétamide, agite 5 minutes, ajoute 52 µl d'iodure de triméthylsilyle, agite 2 heures à température ambiante, ajoute 1,25 ml de tétrahydrofuranne et maintient 1 heure sous agitation. On évapore le solvant sous pression réduite, reprend le résidu par de l'éther, agite 30 minutes et sèche. On ajoute une solution d'éther à 10% d'éthanol, agite 2 heures, filtre et évapore le solvant sous pression réduite. On recueille 68 mg de produit attendu.
Spectre RMN ($CDCl_3$ 300 MHz ppm)

| | |
|---|---|
| 1,43 | $P-CH_3$ |
| 3,40 à 3,80 | $CH_2-S$ |
| 5,60 | $-CH \sim P$ |
| 5,63 | $-\underline{CH}=CH-CH_2-N^+$ |
| 6,33 | $-CH=\underline{CH}-CH_2-N^+$ |
| 6,74-6,76 | $H_5$ du thiazole |
| 6,98 | aryle dichloré |
| 7,17 | $-\underline{CH}=CH-CH_2-N^+$ |
| 7,88-8,14-8,28-9,08-9,22 | thiénopyridine |

**<u>EXEMPLE 11</u> : Sel interne de (6R-(3(E), 6alpha, 7béta(Z))) 1-(3(7-(((2-amino 4-thiazolyl) (((éthoxyméthylphosphinyl) (2,5-dichloro 3,4-dihydroxyphényl) méthoxy) imino) acétyl) amino) 2-carboxy 8-oxo 5-thia 1-azabicyclo[4,2,0]oct-2-èn-3-yl) 2-propényl) quinoléinium (isomère A).**

<u>Stade A</u> : [6R(3(E), 6alpha, 7béta(Z))] iodure de 1-(3-(7-(((((éthoxyméthylphosphinyl) 2,5-dichloro (3,4-bis((2-méthoxyéthoxy) méthoxy) phényl) méthoxy) imino) (2-((triphénylméthyl) amino) 4-thiazolyl) acétyl) amino) 2-(((4-méthoxyphényl) méthoxy) carbonyl) 8-oxo 5-thia 1-azabicyclo-(4.2.0)-oct-2-èn-3-yl) 2-propényl) quinoléinium (isomère A).

On opère comme au stade G de l'exemple 4 en utilisant au départ 2,80 g de produit iodé préparé comme indiqué à l'exemple 8 stade F et 1,36 ml de quinoléine. On obtient 1,34 g de produit attendu. F = 150°C.
Spectre RMN ($CDCl_3$ 300 MHz ppm)

| | |
|---|---|
| 0,97-1,08 | $P-O-CH_2-\underline{CH_3}$ |
| 1,66 | $P-CH_3$ |
| 3,57-3,98 | $P-O-\underline{CH_2}-CH_3$ et $CH_2$ de OMEM |
| 3,30-3,37 | $CH_3$ de OMEM |
| 3,78-3,8 | $\Phi-O-CH_3$ |
| 5,76-5,98-6,25 | $N^+-CH_2-CH=CH$ et $CH \sim P$ |
| 6,46 | $-\underline{CH}=CH-CH_2-N^+$ |

| | |
|---|---|
| 6,76-6,79 | $H_5$ thiazole |
| 7,25 à 7,38 | trityle |
| 7,50-7,59 | dichloroaryle |

Stade B : sel interne de (6R-(3(E), 6alpha, 7béta(Z))) 1-(3-(7-(((2-amino 4-thiazolyl) (((éthoxyméthylphosphinyl) (2,5-dichloro 3,4-dihydroxyphényl) méthoxy) imino) acétyl) amino) 2-carboxy 8-oxo 5-thia 1-azabicyclo[4,2,0] oct-2-èn-3-yl) 2-propényl) quinoléinium (isomère A).

On opère comme au stade H de l'exemple 1 en utilisant au départ 1,4 g du produit obtenu comme au stade A ci-dessus et 14 ml d'acide trifluoroacétique à 10% d'anisole. On obtient 750 mg de produit attendu.

Spectre RMN (DMSO ppm)

| | |
|---|---|
| 1,15 | $P-O-CH_2-\underline{CH_3}$ |
| 1,48 | $P-CH_3$ |
| 3,50 à 4,05 | $S-CH_2$ et $P-O-\underline{CH_2}-CH_3$ |
| 5,71 | CH $\sim$ P |
| 6,42 à 6,98 | $N^+-CH_2-CH$ |
| 6,76 | $H_5$ thiazole |
| 6,98 | dichloroaryle |
| 7,36 | $NH_2$ |
| 8,07-8,26-8,54-9,84 | quinoléine |

**EXEMPLE 12 : Sel interne de (6R-(3(E), 6alpha, 7béta(Z))) 1-(3-(7-(((2-amino 4-thiazolyl) (((éthoxyméthylphonphinyl) (2,5-dichloro 3,4-dihydroxyphényl) méthoxy) imino) acétyl) amino) 2-carboxy 8-oxo 5-thia 1-azabicyclo[4,2,0]oct-2-èn-3-yl) 2-propényl) quinoléinium (isomère B).**

Stade A : [6R(3(E), 6alpha, 7béta(Z))] iodure de 1-(3-(7-(((((éthoxyméthylphosphinyl) 2,5-dichloro (3,4-bis((2-méthoxyéthoxy) méthoxy) phényl) méthoxy) imino) (2-((triphénylméthyl) amino) 4-thiazolyl) acétyl) amino) 2-(((4-méthoxyphényl) méthoxy) carbonyl) 8-oxo 5-thia 1-azabicyclo-(4.2.0)oct-2-èn-3-yl) 2-propényl) quinoléinium (isomère B).

On opère comme au stade G de l'exemple 4 en utilisant au départ 1,55 g de produit iodé préparé comme indiqué à l'exemple 9 stade D et 0,695 ml de quinoléine. On obtient 0,695 g de produit attendu.

Spectre RMN (DMSO ppm)

| | |
|---|---|
| 1,27 et 4,13 | P-OEt |
| 1,27-1,36 | $P-CH_3$ |
| 3,37 | $CH_3$ de OMEM |
| 3,57-3,97-4,13 | $O-CH_2-CH_2-O$ de OMEM et $S-CH_2$ |
| 5,23 | $O-CH_2-O$ de OMEM |
| 5,77-5,97-6,16 | CH $\sim$ P et $N^+-CH_2$ |
| 6,47 | $N^+-CH_2-\underline{CH}$ |

| | |
|---|---|
| 6,74-6,79 | $H_5$ thiazole |
| 6,89-7,37 | $\Phi$-$CH_2$-O |
| 7,29 | trityle |
| 7,97-8,10 à 8,30-8,38-8,98-10,41 | quinoléine |

Stade B : sel interne de (6R-(3(E), 6alpha, 7béta(Z))) 1-(3-(7-(((2-amino 4-thiazolyl) (((éthoxyméthylphosphinyl) (2,5-dichloro 3,4-dihydroxyphényl) méthoxy) imino) acétyl) amino) 2-carboxy 8-oxo 5-thia 1-azabicyclo[4,2,0] oct-2-èn-3-yl) 2-propényl) quinoléinium (isomère B).

On opère comme au stade H de l'exemple 1 en utilisant au départ 0,750 g de produit obtenu comme au stade A ci-dessus et 8 ml d'acide trifluoroacétique à 10% d'anisole. On obtient 539 mg de produit attendu.
Spectre RMN (DMSO ppm)

| | |
|---|---|
| 1,13-1,15 | -$CH_3$ de P-(OEt) |
| 1,53 | P-$CH_3$ |
| 3,70 à 4,0 | $CH_2$ de P-(OEt) |
| 3,50 à 3,8 | S-$CH_2$ |
| 5,68 | CH$\sim$P |
| 5,88 | $N^+$-$CH_2$ |
| 6,41 | $N^+$-$CH_2$-<u>CH</u> |
| 6,74-6,77 | $H_5$ thiazole |
| 6,98 | dichloroaryle |
| 7,30 | $NH_2$ |
| 8,07-8,22 à 8,31-8,53-9,34 | quinoléine |

**EXEMPLE 13 : Sel interne de (6R-(3(E), 6alpha, 7béta(Z))) 1-(3-(7-(((2-amino 4-thiazolyl) (((hydroxyméthylphosphinyl) (2,5-dichloro 3,4-dihydroxyphényl) méthoxy) imino) acétyl) amino) 2-carboxy 8-oxo 5-thia 1-azabicyclo[4,2,0]oct-2-èn-3-yl) 2-propényl) quinoléinium.**

On opère comme à l'exemple 10 en utilisant 80 mg de produit obtenu à l'exemple 11 ou 12 ou leur mélange, 88 µl de N-méthyl N-triméthylsilyl trifluoroacétamide puis 52 µl d'iodure de triméthylsilyle. On obtient 68 mg de produit attendu.
Spectre RMN ($CDCl_3$ ppm)

| | |
|---|---|
| 1,00 | P-$CH_3$ |
| 3,59 à 3,79 | S-$CH_2$ |
| 5,62 | O-CH-P |
| 6,41 | $N^+$-$CH_2$-<u>CH</u> |
| 6,79 | $H_5$ thiazole |
| 6,95 à 7,20 | dichloroaryle et $N^+$-$CH_2$-CH=<u>CH</u> |

8,27-8,53-8,07-9,58 quinoléine

En plus des produits décrits ci-dessus dans les exemples, les produits répondant à la formule ci-dessous et résultant des combinaisons des différentes valeurs des substituants représentées dans les tableaux qui suivent, constituent des produits pouvant être obtenus selon l'invention.

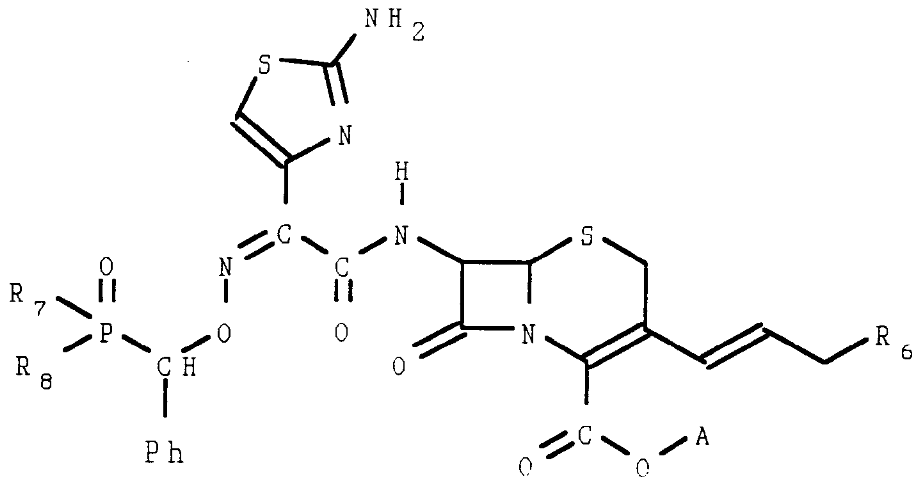

Dans ces produits, $R_7/R_8$ représentent OH/OH, OEt/OEt, $CH_3$/OH et $CH_3$/OEt.

| Ph | $R_6$ | $R_7$ | $R_8$ |
|---|---|---|---|
| 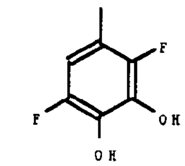 | 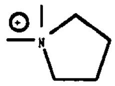 | OH | OH |
| 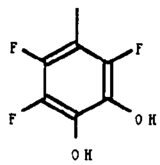 | 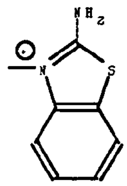 | OEt | OEt |
| 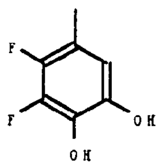 | 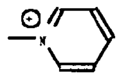 | CH3 | |
| 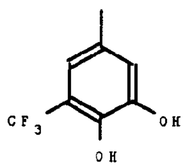 | 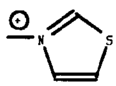 | | |
| 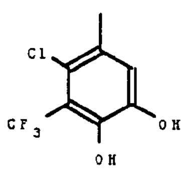 | 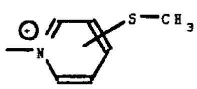 | | |
| 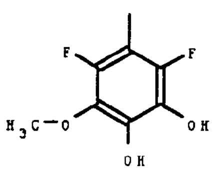 | 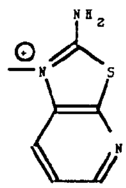 | | |
| | 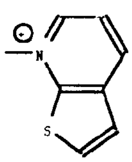 | | |

| Ph | $R_6$ | $R_7$ | $R_8$ |
|---|---|---|---|
| 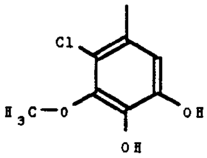<br>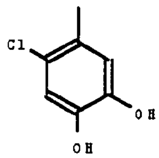<br>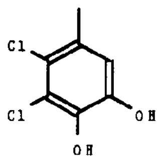<br>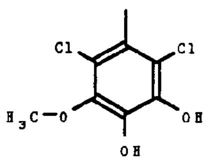<br>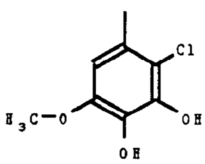<br>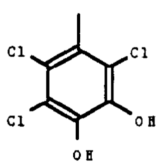 | 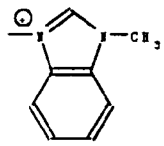<br>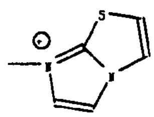<br>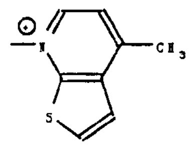<br>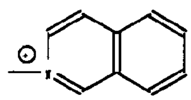<br>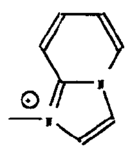<br>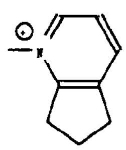 | OH<br>OEt<br>CH3 | OH<br>OEt |

| Ph | $R_6$ | $R_7$ | $R_8$ |
|---|---|---|---|
| 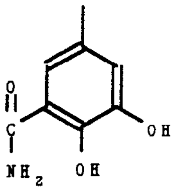<br>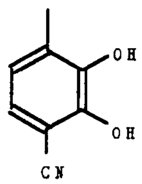<br>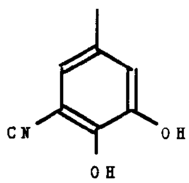 | 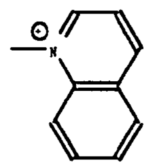<br>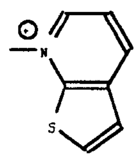<br>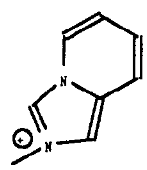<br>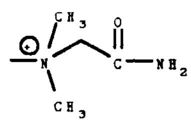<br>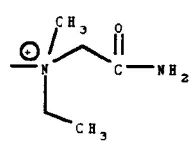 | OH<br>OEt<br>CH3 | OH<br>OEt |

**<u>EXEMPLE 14</u> : On a réalisé des préparations pour injections de formule :**

- Produit de l'exemple 4 500 mg
- Excipient aqueux stérile q.s.p. 5 $cm^3$

**<u>ETUDE PHARMACOLOGIQUE DES PRODUITS DE L'INVENTION</u>**

**Activité in vitro, méthode des dilutions en milieu liquide.**

On prépare une série de tubes dans lesquels on répartit une même quantité de milieu nutritif stérile. On distribue dans chaque tube des quantités croissantes du produit à étudier, puis chaque tube est ensemencé avec une souche

bactérienne. Après incubation de vingt-quatre heures à l'étuve à 37°C, l'inhibition de la croissance est appréciée par transillumination ce qui permet de déterminer les concentrations minimales inhibitrices (C.M.I.) exprimées en µg/$cm^3$ (TABLEAU I).

**Activité in vitro, méthode des dilutions en milieu solide.**

On prépare une série de boîtes dans lesquelles on répartit une même quantité de milieu nutritif stérile, contenant des quantités croissantes du produit à étudier puis chaque boîte est ensemencée avec plusieurs souches bactériennes.

Après incubation de 24 heures en étuve à 37°C, l'inhibition de la croissance est appréciée par l'absence de tout développement bactérien ce qui permet de déterminer les concentrations minimales inhibitrices (CMI) exprimées en microgrammes/$cm^3$.

Les résultats sont exprimés sous forme de moyennes géométriques de toutes les CMI obtenues, c'est-à-dire les $CMI_{50}$ et $CMI_{90}$, lesquelles représentent les concentrations minimum d'antibiotiques permettant d'inhiber la croissance de 50 et 90 % des souches étudiées.

On a obtenu les résultats suivants :

| Produit de l'exemple | Staphylocoques Pénicilline résistants | Enterobactéries Céfotaxime résistantes (35 souches) | Enterobactéries Céfotaxime sensibles (26 souches) | Pseudomonas aeruginosa (36 souches) |
|---|---|---|---|---|
| 1 | 0,35 | 1,17 | 0,22 | - |
| 2 | 0,21 | 1,02 | 0,14 | - |
| 3 | 0,88 | 1,68 | 0,3 | - |
| 4 | 0,42 | 0,26 | 0,098 | 0,96 |
| 5 | 0,11 | 1,06 | 0,071 | - |
| 6 | 0,25 | 2,89 | 0,33 | - |
| 7 | 0,11 | 0,96 | 0,087 | - |
| 8 | 0,57 | 0,24 | - | 0,26 |
| 10 | 0,91 | 0,64 | - | 0,21 |
| 11 | 0,57 | 0,26 | - | 0,24 |
| 12 | 0,68 | 0,29 | - | 0,29 |
| 13 | 0,78 | 0,25 | - | 0,091 |

**Revendications**

**1)** Les produits de formule générale (I) :

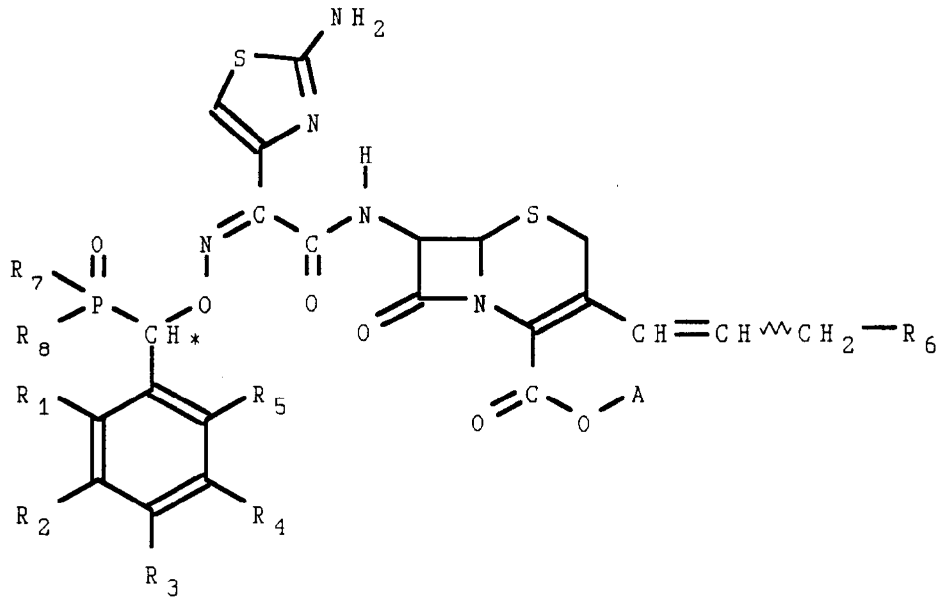

**(I)**

isomère <u>syn</u>, sous forme (*), (R) ou (S) ou d'un mélange (R,S), sous forme de sels internes ou de sels avec les acides minéraux ou organiques ou avec les bases, formule dans laquelle :

$R_1$, $R_2$, $R_3$ et $R_5$ identiques ou différents représentent un atome d'hydrogène, un atome d'halogène ou un des radicaux choisis parmi le radical hydroxy, alkyle renfermant de 1 à 4 atomes de carbone, éventuellement substitué par un ou plusieurs atomes d'halogène, alkyloxy renfermant de 1 à 4 atomes de carbone, mercapto, alkylthio renfermant de 1 à 4 atomes de carbone, nitro, cyano, amino, alkylamino renfermant de 1 à 4 atomes de carbone, dialkylamino renfermant de 2 à 8 atomes de carbone, carbamoyle, (alkylamino) carbonyle renfermant de 2 à 5 atomes de carbone, (dialkylamino) carbonyle renfermant de 3 à 9 atomes de carbone, carboxy, alcoxycarbonyle renfermant de 2 à 5 atomes de carbone, acyloxy renfermant de 1 à 8 atomes de carbone,

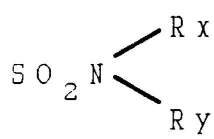

dans lequel Rx et Ry identiques ou différents représentent un atome d'hydrogène ou un radical alkyle renfermant de 1 à 4 atomes de carbone,

$R_4$ représente un radical hydroxy ou un radical acyloxy renfermant de 1 à 8 atomes de carbone,

$R_7$ représente un radical alkyle renfermant de 1 à 4 atomes de carbone, un radical hydroxy, un radical alkyloxy renfermant de 1 à 4 atomes de carbone ou un radical phényl,

$R_8$ représente un radical hydroxy ou un radical alkyloxy renfermant de 1 à 4 atomes de carbone,

ou $R_7$ et $R_8$ représentent ensemble un radical alkylène dioxy renfermant de 2 à 8 atomes de carbone,

A représente un atome d'hydrogène, un équivalent de métal alcalin, alcalino-terreux, de magnésium, d'ammonium ou d'une base organique aminée ou A représente le reste d'un groupement ester facilement clivable, ou bien $CO_2A$ représente $CO_2^{\ominus}$, le trait ondulé signifie que le groupement $CH_2R_6$ peut se trouver dans la position E ou Z et $R_6$ représente sous forme d'ammonium quaternaire, un des radicaux suivants :

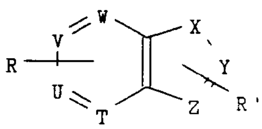
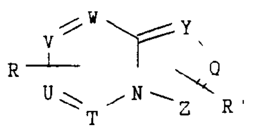
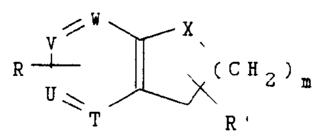
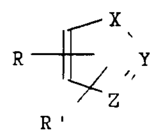
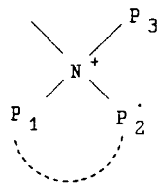
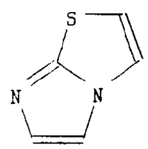
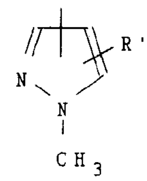
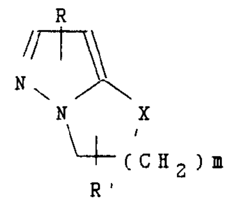
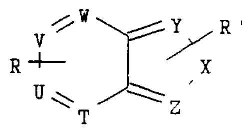
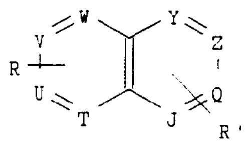
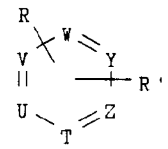
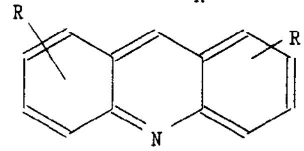

dans lesquels X représente $CH_2$, NH, O ou S ;
Q, J, Y, T, U, V, W et Z identiques ou différents représentent indépendamment les uns des autres CH ou N, étant entendu que chacun de ces radicaux cycliques contient de 1 à 5 hétéroatomes, qu'au moins un de ces hétéroatomes est l'atome d'azote et que ces radicaux cycliques peuvent être substitués par un ou plusieurs radicaux R ou R' ;
R et R', identiques ou différents, représentent un atome d'hydrogène, un radical alkyle renfermant de 1 à 4 atomes de carbone, un radical alkyloxy renfermant de 1 à 4 atomes de carbone, un atome d'halogène, un radical cyano, un radical $CO_2$-$Q_1$, CO-$NQ_1(Q_2)$, $NQ_1(Q_2)$, $SO_2$-$NQ_1(Q_2)$, CS-$NH_2$, NH-CO-$Q_1$, CH=N-OH, CH=N-O-$Q_1$, $CH_2$-CN, $CH_2$-S-$Q_1$, $SQ_1$, dans lesquels $Q_1$ et $Q_2$, identiques ou différents, représentent un atome d'hydrogène ou un radical alkyle renfermant de 1 à 4 atomes de carbone, $P_1$, $P_2$ et $P_3$, identiques ou différents, représentent un radical alkyle renfermant de 1 à 4 atomes de carbone, éventuellement substitué par un des substituants indiqués ci-dessus pour R et R', ou encore, pour l'un d'entre eux, par un radical hydroxy, le trait pointillé indique que $P_1$ et $P_2$ peuvent éventuellement former avec l'atome d'azote auquel ils sont liés, un hétérocycle à 5 ou 6 chaînons et m représente 1, 2 ou 3.

**2)** les produits de formule générale (I) telle que définie à la revendication 1 dans laquelle $R_6$ représente un des radicaux suivants :

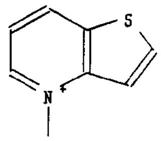 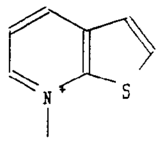 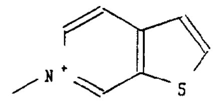 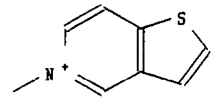

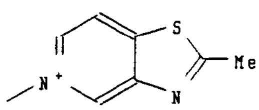 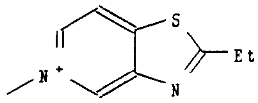 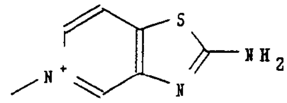

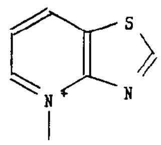 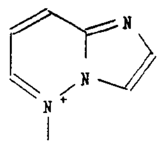 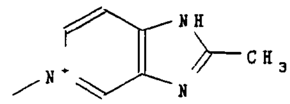

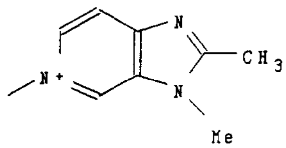 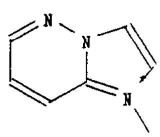 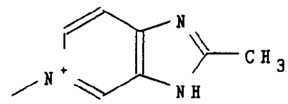

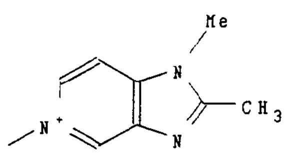 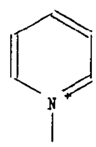 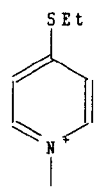 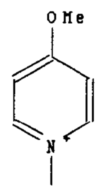

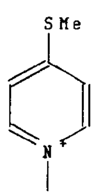 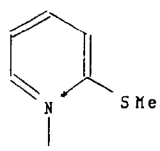 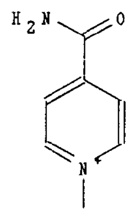 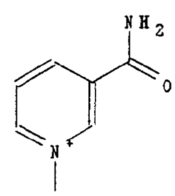

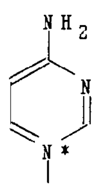 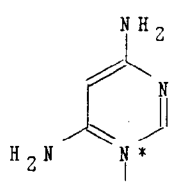 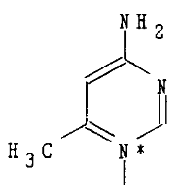 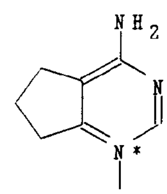

ou encore l'un des radicaux suivants :

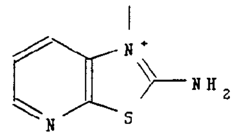 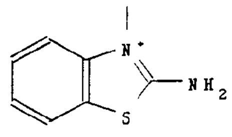 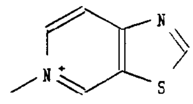

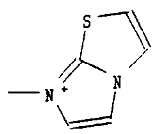 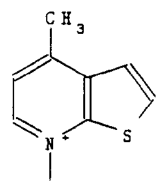 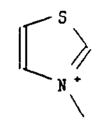 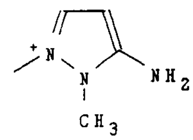

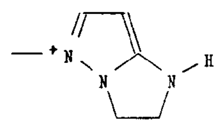 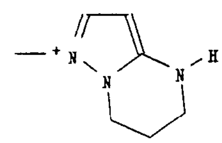

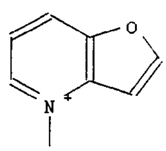 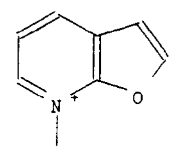 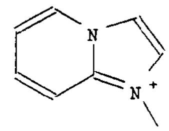 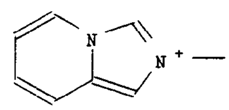

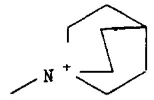 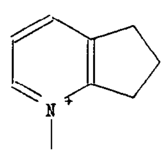 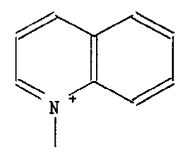

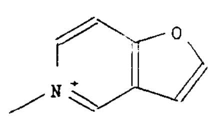 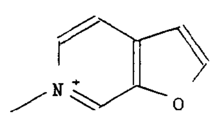

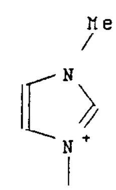 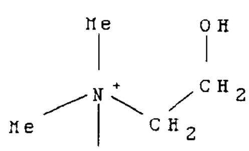 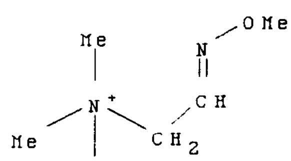

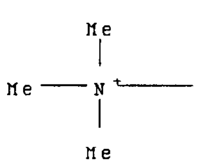 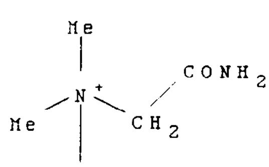 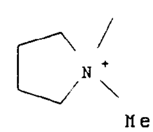

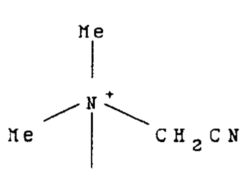 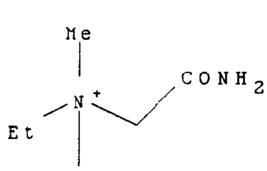 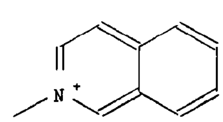

**3)** Les produits de formule (I) telle que définie à l'une des revendications 1 et 2, dans laquelle $R_6$ représente le

radical quinoléinium, isoquinoléinium, 4-(méthylthio) pyridinium, thièno[2,3-b]pyridinium, 1-méthyl pyrrolidinium, N-méthyl N-éthyl N-(2-amino 2-oxoéthyl) aminium, imidazo(1,2-a)pyridinium ou le radical 6,7-dihydro-5H-pyrindinium.

**4)** Les produits de formule générale (I) telle que définie à l'une quelconque des revendications 1, 2 ou 3 dans laquelle $R_3$ et $R_4$ représentent chacun un radical hydroxy.

**5)** Les produits de formule générale (I) telle que définie à l'une quelconque des revendications 1, 2, 3 ou 4 dans laquelle $R_2$ et $R_5$ représentent chacun un atome de chlore.

**6)** Les produits de formule générale (I) telle que définie à l'une quelconque des revendications 1, 2, 3 ou 4 dans laquelle $R_2$ et $R_5$ représentent chacun un atome de fluor.

**7)** Les produits de formule générale (I) telle que définie à l'une quelconque des revendications 1, 2, 3 ou 4 dans laquelle $R_1$ et $R_2$ représentent chacun un atome de fluor.

**8)** Les produits de formule générale (I) telle que définie à l'une quelconque des revendications 1, 2, 3 ou 4 dans laquelle $R_2$ représente un radical méthoxy et l'un de $R_1$ ou $R_5$ représente un atome de chlore.

**9)** Les produits de formule générale (I) dont les noms suivent :

- le sel interne de (6R-(3-(E) 6alpha, 7béta-(Z))) 1-(3-(7-(((2-amino 4-thiazolyl) ((éthoxyméthylphosphinyl (3,4-dihydroxyphényl) méthoxy) imino) acétyl) amino) 2-carboxy 8-oxo 5-thia 1-azabicyclo[4,2,0]oct-2-èn-3-yl) 2-propényl) quinoléinium,

- le sel interne de (6R-(3-(E) 6alpha, 7béta-(Z))) 7-(3-(7-(((2-amino 4-thiazolyl) ((éthoxyméthylphosphinyl (3,4-dihydroxyphényl) méthoxy) imino) acétyl) amino) 2-carboxy 8-oxo 5-thia 1-azabicyclo[4,2,0]oct-2-èn-3-yl) 2-propényl) thiéno(2,3-b)pyridinium,

- le sel interne de (6R-(3-(E) 6alpha, 7béta-(Z))) 7-(3-(7-(((2-amino 4-thiazolyl) ((hydroxyméthylphosphinyl (3,4-dihydroxyphényl) méthoxy) imino) acétyl) amino) 2-carboxy 8-oxo 5-thia 1-azabicyclo[4,2,0]oct-2-èn-3-yl) 2-propényl) thiéno(2,3-b)pyridinium,

- le sel interne de (6R-(3-(E) 6alpha, 7béta-(Z))) 7-(3-(7-(((2-amino 4-thiazolyl) (((2,5-dichloro 3,4-dihydroxyphényl) (diéthoxyphosphinyl) méthoxy) imino) acétyl) amino) 2-carboxy 8-oxo 5-thia 1-azabicyclo[4,2,0] oct-2-èn-3-yl) 2-propényl) thiéno(2,3-b)pyridinium,

- le sel interne de (6R-(3-(E) 6alpha, 7béta-(Z))) 7-(3-(7-(((2-amino 4-thiazolyl) (((2,5-dichloro 3,4-dihydroxyphényl) phosphonométhoxy) imino) acétyl) amino) 2-carboxy 8-oxo 5-thia 1-azabicyclo[4,2,0]oct-2-èn-3-yl) 2-propényl) thiéno(2,3-b)pyridinium,

- le sel interne de (6R-(3(E), 6alpha, 7béta(Z))) 7-(3-(7-(((2-amino 4-thiazolyl) ((diéthoxyphosphinyl (3,4-dihydroxyphényl) méthoxy) imino) acétyl) amino) 2-carboxy 8-oxo 5-thia 1-azabicyclo[4,2,0]oct-2-èn-3-yl) 2-propényl) thiéno(2,3-b)pyridinium,

- le sel interne de (6R-(3(E), 6alpha, 7béta(Z))) 7-(3-(7-(((2-amino 4-thiazolyl) ((3,4-dihydroxyphényl) phosphonométhoxy) imino) acétyl) amino) 2-carboxy 8-oxo 5-thia 1-azabicyclo[4,2,0]oct-2-èn-3-yl) 2-propényl) thiéno (2,3-b)pyridinium,

- le sel interne de (6R-(3(E), 6alpha, 7béta(Z))) 7-(3-(7-(((2-amino 4-thiazolyl) (((éthoxyméthylphosphinyl) (2,5-dichloro 3,4-dihydroxyphényl) méthoxy) imino) acétyl) amino) 2-carboxy 8-oxo 5-thia 1-azabicyclo[4,2,0] oct-2-èn-3-yl) 2-propényl) thiéno(2,3-b)pyridinium (isomère A) et (isomère B),

- le sel interne de (6R-(3(E), 6alpha, 7béta(Z))) 7-(3-(7-(((2-amino 4-thiazolyl) (((hydroxyméthylphosphinyl) (2,5-dichloro 3,4-dihydroxyphényl) méthoxy) imino) acétyl) amino) 2-carboxy 8-oxo 5-thia 1-azabicyclo[4,2,0] oct-2-èn-3-yl) 2-propényl) thiéno(2,3-b)pyridinium,

- le sel interne de (6R-(3(E), 6alpha, 7béta(Z))) 1-(3-(7-(((2-amino 4-thiazolyl) (((éthoxyméthylphosphinyl) (2,5-dichloro 3,4-dihydroxyphényl) méthoxy) imino) acétyl) amino) 2-carboxy 8-oxo 5-thia 1-azabicyclo[4,2,0] oct-2-èn-3-yl) 2-propényl) quinoléinium (isomère A) et (isomère B),

- le sel interne de (6R-(3(E), 6alpha, 7béta(Z))) 1-(3-(7-(((2-amino 4-thiazolyl) (((hydroxyméthylphosphinyl) (2,5-dichloro 3,4-dihydroxyphényl) méthoxy) imino) acétyl) amino) 2-carboxy 8-oxo 5-thia 1-azabicyclo[4,2,0] oct-2-èn-3-yl) 2-propényl) quinoléinium.

**10)** Procédé de préparation des produits de formule (I) telle que définie à la revendication 1, caractérisé en ce que l'aldéhyde aromatique de formule (II) :

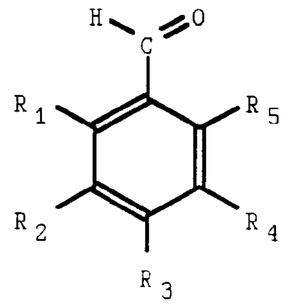

**(II)**

dans laquelle $R_1$, $R_2$, $R_3$, $R_4$ et $R_5$ sont tels que définis ci-dessus, est si nécessaire protégé en ses fonctions réactives et transformé ainsi en aldéhyde aromatique de formule ($II_p$) :

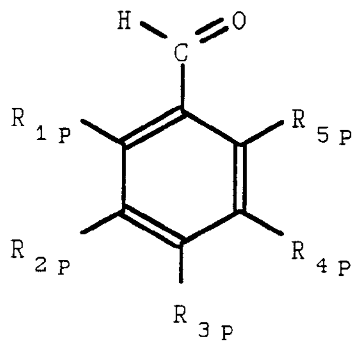

**($II_p$)**

dans laquelle $R_{1p}$, $R_{2p}$, $R_{3p}$, $R_{4p}$ et $R_{5p}$ représentent respectivement les valeurs de $R_1$, $R_2$, $R_3$, $R_4$ et $R_5$ telles que définies précédemment ou une fonction réactive protégée, ledit aldéhyde de formule ($II_p$) est traité par un réactif de formule (III) :

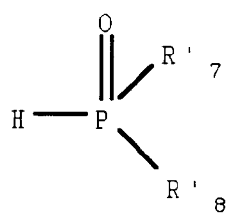

**(III)**

dans laquelle $R'_7$ représente un radical alkyle renfermant de 1 à 4 atomes de carbone, un radical alkyloxy renfermant de 1 à 4 atomes de carbone ou un radical phényle, $R'_8$ représente un radical alkyloxy renfermant de 1 à 4 atomes de carbone ou $R'_7$ et $R'_8$ représentent ensemble un radical alkylènedioxy renfermant de 2 à 8 atomes de carbone, en présence d'une base, pour obtenir le composé de formule (IV) :

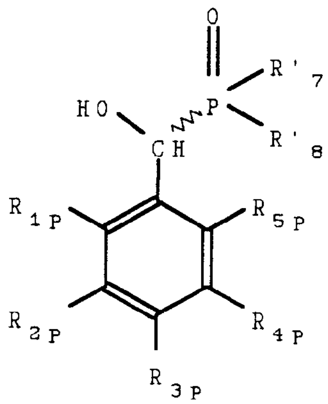

(IV)

dans laquelle $R'_7$ et $R'_8$ sont définis comme précédemment et le trait ondulé symbolise un mélange d'isomères que si désiré l'on sépare, et produit de formule (IV) sous forme d'isomère A, d'isomère B ou de mélange, que l'on traite par le N-hydroxy phtalimide, le cas échéant en présence d'un agent activant, pour obtenir le dérivé de formule (V) :

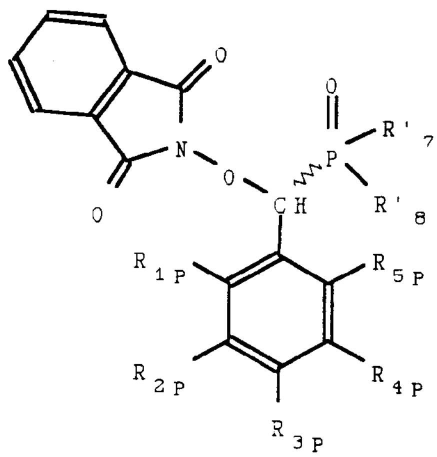

(V)

sous forme d'isomère A, d'isomère B ou de mélange que l'on hydrolyse en hydroxylamine O-substituée de formule (VI) :

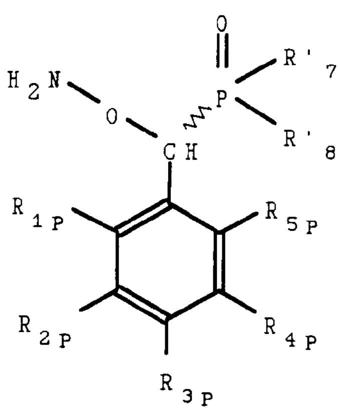

(VI)

sous forme d'isomère A, d'isomère B ou de mélange que l'on condense avec un dérivé de l'acide 2-(2-amino thiazol-4-yl) 2-oxo acétique de formule (VII) :

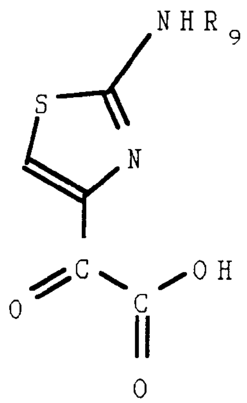

(VII)

dans laquelle $R_9$ représente un atome d'hydrogène ou un groupe protecteur de la fonction amine, pour former le dérivé de l'acide alpha-alkoxyimino acétique "syn" de formule (VIII) :

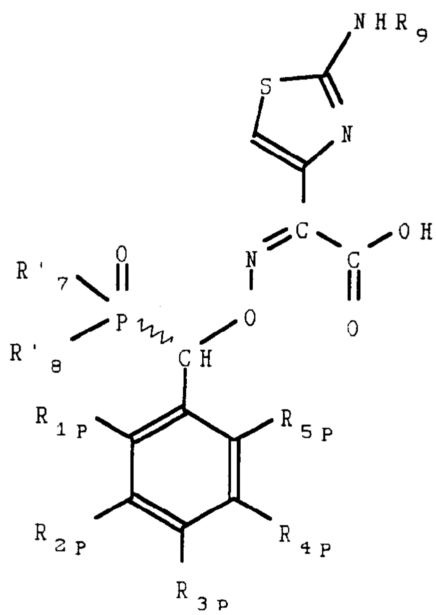

(VIII)

sous forme d'isomère A, d'isomère B ou de mélange dont on prépare le cas échéant, un dérivé fonctionnel, produit de formule (VIII) ou dérivé fontionnel que l'on amidifie avec un ester du chlorhydrate de l'acide 7-amino 3-(3-halo 1-propényl) 8-oxo 5-thia 1-azabicyclo[4,2,0]oct-2-èn-2-carboxylique de formule (IX) :

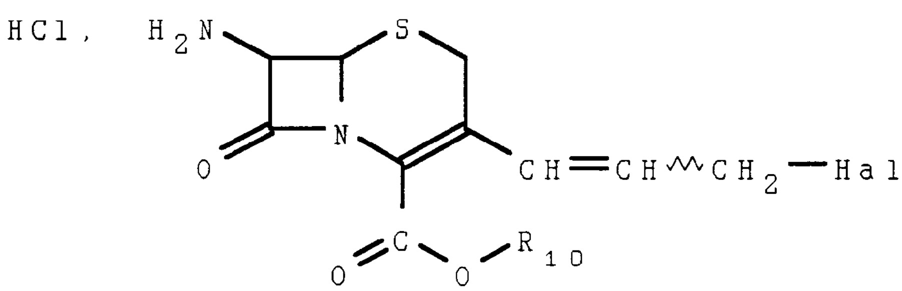

(IX)

ou de ses sels, dans laquelle $R_{10}$ représente le reste d'un ester aisément clivable, pour conduire au dérivé de l'acide 7-(N-substitué amido) 3-(3-halo 1-propényl) 8-oxo 5-thia 1-azabicyclo[4,2,0]oct-2-èn-2-carboxylique de formule (X) :

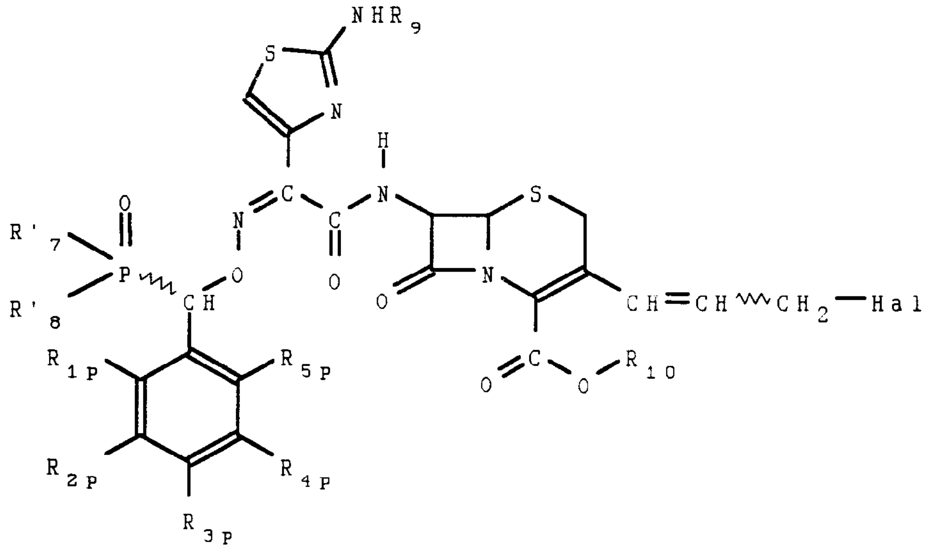

(X)

sous forme d'isomère A, d'isomère B ou de mélange que l'on transforme, le cas échéant, en analogue 3-(3-iodo propényle) de formule (XI) :

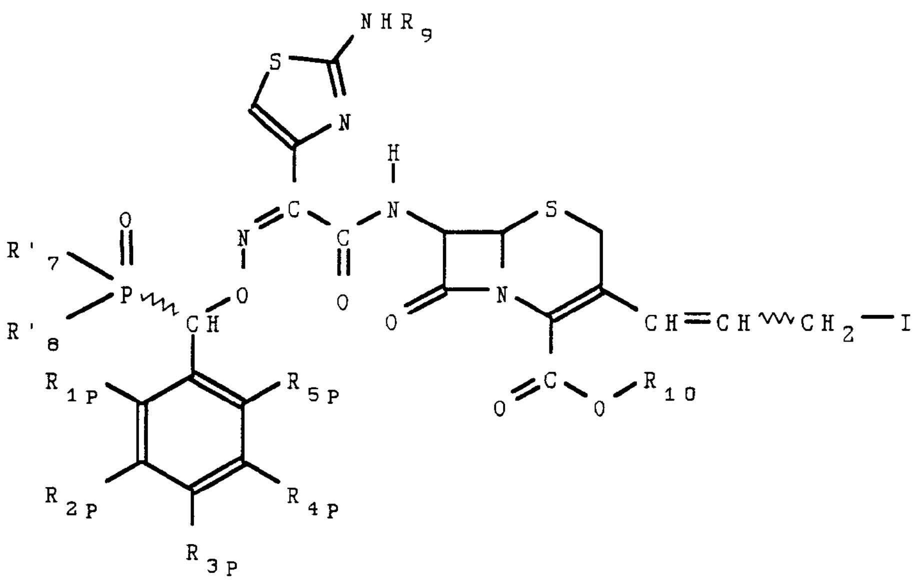

(XI)

sous forme d'isomère A, d'isomère B ou de mélange que l'on traite avec une base de formule $R_6$ pour obtenir le produit de formule (XII) :

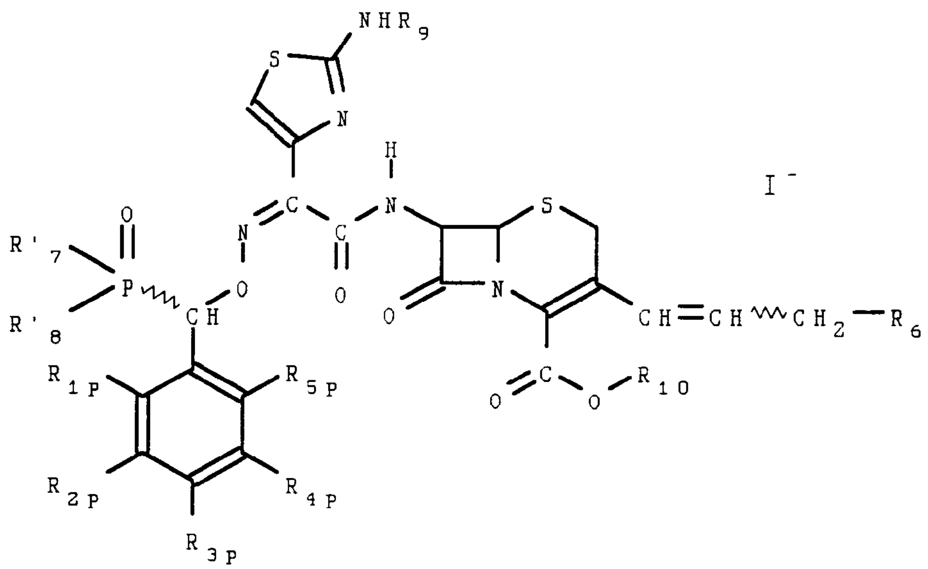

**(XII)**

sous forme d'isomère A, d'isomère B ou de mélange, produit de formule (XII) à partir duquel si désiré, l'on isole les isomères (E) ou (Z) ou transforme les isomères (Z) en isomère (E), produit de formule (XII) que l'on soumet le cas échéant à une ou plusieurs des réactions suivantes, dans un ordre approprié :

a) coupure par hydrolyse ou par action de la thiourée de tout ou partie des groupements esters ou des groupements de protection du radical amino ou des radicaux hydroxyles,

b) déalkylation d'un ou de deux groupements alkyloxy portés par l'atome de phosphore,

c) estérification ou salification par une base du ou des radicaux carboxyliques et salification par une base du ou des radicaux hydroxy portés par l'atome de phosphore,

d) salification par un acide du radical amino,

e) séparation des produits sous forme de mélange R,S en R et S.

**11)** Variante du procédé selon la revendication 10, caractérisé en ce que l'hydroxylamine O-substituée de formule (VI) est condensée au produit de formule (XIII)**:**

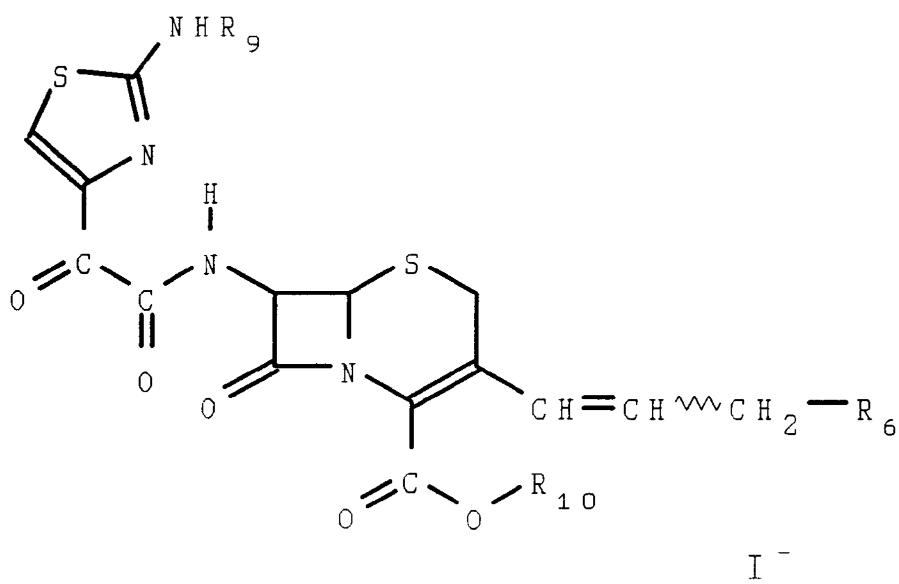

**(XIII)**

pour conduire au produit de formule (XII) telle que définie à la revendication 10.

**12)** A titre de médicaments, les produits répondant à la formule (I) telle que définie à la revendication 1, ainsi que leurs sels d'acides, pharmaceutiquement acceptables.

**13)** A titre de médicaments, les produits tels que définis à l'une quelconque des revendications 2 à 9 ainsi que leurs sels d'acides, pharmaceutiquement acceptables.

**14)** Compositions pharmaceutiques contenant, à titre de principe actif, au moins un médicament selon l'une des revendications 12 ou 13.

**15)** A titre de produits industriels nouveaux, les produits de formules (IV), (V), (VI), (VIII), (X), (XI) et (XII) telles que définies à la revendication 10.

Office européen des brevets

# RAPPORT DE RECHERCHE EUROPEENNE

Numero de la demande
EP 95 40 1699

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | GB-A-2 071 664 (E.R. SQUIBB & SONS INC.)<br>* le document en entier *<br>--- | 1-14 | C07F9/6561<br>A61K31/675<br>C07F9/553<br>C07F9/6539<br>C07F9/40 |
| A | US-A-3 887 549 (BURTON G. CHRISTENSEN)<br>* le document en entier *<br>--- | 1-14 | |
| X | EP-A-0 024 611 (BAYER AG)<br>* exemple 2 *<br>--- | 15 | |
| X | EP-A-0 071 805 (BAYER AG)<br>* exemple 3 *<br>--- | 15 | |
| X | ZH. OBSHCH. KHIM. (ZOKHA4,0044460X);92; VOL.62 (3); PP.583-8, CHUVASH. GOS. UNIV.;CHEBOKSARY; RUSSIA (RU)<br>Kormachev V V et al 'Synthesis of phosphorus-containing esters of 3-(2,2-dichlorovinyl)-2,2-dimethylcyclopropanecarboxylic acid'<br>* le document en entier *<br>--- | 15 | |
| | | | **DOMAINES TECHNIQUES RECHERCHES (Int.Cl.6)** |
| X | CHEMICAL ABSTRACTS, vol. 113, no. 21, 19 Novembre 1990, Columbus, Ohio, US; abstract no. 191466,<br>NAPOLITANO E ET AL 'Regiospecifically substituted deoxybenzoins by Horner condensation of aromatic aldehydes with O-protected.alpha.-aryl-.alpha.-hydroxymethanephosphonates'<br>* abrégé *<br>& GAZZ. CHIM. ITAL. (GCITA9,00165603);89; VOL.119 (1); PP.19-22, UNIV. PISA;FAC. FARM.; PISA; I-56100; ITALY (IT)<br>---<br>-/-- | 15 | C07F<br>A61K |

Le présent rapport a été établi pour toutes les revendications

| Lieu de la recherche | Date d'achèvement de la recherche | Examinateur |
|---|---|---|
| LA HAYE | 3 Novembre 1995 | Beslier, L |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numero de la demande
EP 95 40 1699

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | CHEMICAL ABSTRACTS, vol. 110, no. 11, 13 Mars 1989, Columbus, Ohio, US; abstract no. 094838, NAPOLITANO E ET AL '.alpha.-Aryl-.alpha.-(2-tetrahydropyranyloxy)methanephosphonates as reagents in the Horner reaction. A simple novel synthesis of (.+-.)-combretastatin' * abrégé * & GAZZ. CHIM. ITAL. (GCITA9,00165603);88; VOL.118 (5); PP.415-16, FAC. FARM.;IST. CHIM. ORG.; PISA; I-56100; ITALY (IT) --- | 15 | |
| X | CHEMICAL ABSTRACTS, vol. 089, no. 15, 9 Octobre 1978, Columbus, Ohio, US; abstract no. 129601, STREPIKHEEV Y A ET AL 'Synthesis of substituted.alpha.-hydroxybenzyldialkyl phosphonates and their derivatives' * abrégé * & DEPOSITED DOC. (D8DEP2);76; (VINITI 2264-76,); 21 PP., MOSK. KHIM.-TEKHNOL. INST.;MOSCOW; USSR --- | 15 | DOMAINES TECHNIQUES RECHERCHES (Int.Cl.6) |
| X | CHEMICAL ABSTRACTS, vol. 079, no. 26, 31 Décembre 1973, Columbus, Ohio, US; abstract no. 150691, DAS K G ET AL 'Simple cleavage versus rearrangement reactions in some p- and m-substituted.alpha.-hydroxyalkyl phosphonates' * abrégé * & INDIAN J. CHEM. (IJOCAP);73; VOL.11 (6); PP.552-3, NATL. CHEM. LAB.;POONA; INDIA --- -/-- | 15 | |

Le présent rapport a été établi pour toutes les revendications

| Lieu de la recherche | Date d'achèvement de la recherche | Examinateur |
|---|---|---|
| LA HAYE | 3 Novembre 1995 | Beslier, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

RAPPORT DE RECHERCHE EUROPEENNE

Numero de la demande
EP 95 40 1699

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | CHEMICAL ABSTRACTS, vol. 081, no. 9, 2 Septembre 1974, Columbus, Ohio, US; abstract no. 049744, VASIL'EVA V P ET AL 'Phosphorylation of lignin model compounds with dimethyl phosphite' * abrégé * & SB. TR. MOLODYKH UCH., TOMSK. POLITEKH. INST. (D8MMYC);73; VOL.NO. 1,; PP.166-8, USSR --- | 15 | |
| X | CHEMICAL ABSTRACTS, vol. 077, no. 1, 3 Juillet 1972, Columbus, Ohio, US; abstract no. 005574, TEWARI R S ET AL 'Organophosphorus compounds addition reaction of O,O-dialkyl hydrogen phosphites with substituted aromatic and long chain aliphatic aldehydes' * abrégé * & LABDEV, PART A (LAPSBF);71; VOL.9 (2); PP.112-16, H. B. TECHNOL. INST.;DEP. CHEM.; KANPUR; INDIA --- | 15 | DOMAINES TECHNIQUES RECHERCHES (Int.Cl.6) |
| X | CHEMICAL ABSTRACTS, vol. 077, no. 23, 4 Décembre 1972, Columbus, Ohio, US; abstract no. 152288, SHUKLA R ET AL 'Organophosphorus insecticides. Synthesis of a.alpha.-hydroxy phenyl phosphonates' * abrégé * & INDIAN J. APPL. CHEM. (IJACAN);71; VOL.34 (6); PP.249-52, H. B. TECHNOL. INST.;DEP. CHEM.; KANPUR; INDIA ----- | 15 | |

Le présent rapport a été établi pour toutes les revendications

| Lieu de la recherche | Date d'achèvement de la recherche | Examinateur |
|---|---|---|
| LA HAYE | 3 Novembre 1995 | Beslier, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)